# EUROPEAN PATENT APPLICATION

(11) **EP 1 548 101 A1**
(43) Date of publication of application: **29.06.2005**
(21) Application number: 03794227.3
(22) Date of filing: 04.09.2003
(51) Int. Cl.: C12N 7/00, C12N 15/867

(54) **METHODS OF PRODUCING A VIRAL VECTOR COMPRISING A MEMBRANE PROTEIN THAT BINDS TO SIALIC ACID AS A COMPONENT OF THE ENVELOPE USING NEURAMINIDASE DERIVED FROM GRAM-POSITIVE BACTERIA**

(30) Priority: 04.09.2002 JP 2002258576
(71) Applicant: Dnavec Research Inc., Tsukuba-shi, Ibaraki 305-0856 (JP)
(72) Inventor: KOBAYASHI, Masanori c/o Disc. Res.Lab.,Shionogi&Co, Settsu-shi, Osaka 566-0022 (JP); UEDA, Yasuji c/o DNAVEC Research Inc., Tsukuba-shi, Ibaraki 305-0856 (JP); HASEGAWA, Mamoru c/o DNAVEC Research Inc., Tsukuba-shi, Ibaraki 305-0856 (JP)
(74) Representative: VOSSIUS & PARTNER
(86) International application number: PCT/JP2003/011299
(87) International publication number: WO 2004/022731

(57) **Abstract**

The present invention provides methods for producing a viral vector comprising a membrane protein that binds to sialic acid as a component of the envelope, using neuraminidase (NA) derived from Gram-positive bacteria. The methods comprise the steps of culturing cells producing a viral vector in the presence of an NA from Gram-positive bacteria, and recovering the produced virus. The methods of this invention enable the production of high titer virus at high cost performance. Such a viral vector is capable of transferring genes at high efficiency into cells such as blood cells and hematopoietic cells, including hematopoietic stem cells, and mucous cells including mucoepithelial cells, those not amenable to gene transfer by conventional methods, and therefore should be useful as a vector for gene therapy.

## Description

### Technical Field

The present invention relates to methods for producing a viral vector comprising a membrane protein that binds to sialic acid as a component of the envelope, using neuraminidase derived from Gram-positive bacteria.

### Background Art

Glycoproteins containing sialic acid are present on the surface of most cells. Certain types of viruses possess a protein capable of binding to such sialic acid as a component of the envelope, which facilitates virus particles' adherence to the cells. For example, the influenza virus binds to sialic acid present on the cell surface through an envelope protein called hemagglutinin (HA). However, the binding to sialic acid on the host cell surface needs to be dissociated upon budding in viral replication steps, a process in which the neuraminidase protein (NA) of the influenza virus plays an important role. In addition, NA is reported to play a role in the inhibition of self-aggregation (Compans R.W. *et al*. J. Virol. 4: 528-534 (1969); Palese P. *et al*. Virology 61: 397-410 (1974); Griffin J.A. *et al*. Virology 125: 324-334 (1983); Liu C. *et al*. J. Virol. 69: 1099-1106 (1995)).

The binding activity of the HA protein has been recognized and appropriated in the preparation of HA pseudotyped viruses having improved gene transfection capacity. However, these pseudotyped viruses are characterized by a low viral titer without the inclusion of neuraminidase (Hatziioannou T. *et al*. J. Virol. 72: 5313-5317 (1998)). By exogenously supplying NA to the vector producing system, it is possible to promote the incorporation of the HA protein into virion and thus increase the titer (Dong J. *et al*. J. Virol. 66: 7374-7382 (1992); Negre D. *et al*. Gene Ther. 7: 1613-1623 (2000); Morse K.M. *et al*. The American Society of Gene Therapy's 5th Annual Meeting (2002); WO01/92508). Nevertheless, to date, the titer of HA pseudotyped viruses produced using purified NA currently available is not satisfactory.

### Disclosure of the Invention

An objective of the present invention is to provide methods for producing a viral vector containing a membrane protein that binds to sialic acid as a component of the envelope, using neuraminidase derived from Gram-positive bacteria.

The conventional methods for the production of an HA pseudotyped viruses often use NA from *Vibrio cholerae,* which is categorized as Gram-negative bacteria. However, the titer of the vector produced using NA from *Vibrio cholerae* is low. Furthermore, it remains a challenge to produce such vectors in a large scale industrially. To develop a technology for producing a viral vector containing a membrane protein that binds to sialic acid as a component of the envelope not only at high titer but also at low cost, the present inventors searched for a novel NA that could be used for virus production. The present inventors discovered that, by using NA derived from Gram-positive bacteria, they could successfully produce a virus having significantly higher titer as compared to those produced with NA from *Vibrio cholerae*. Accordingly, the use of NA from Gram-positive bacteria should enable the industrial production of pseudotyped vectors in large scale in an efficient and economical way.

Thus, the present invention relates to methods of producing a viral vector containing a membrane protein that binds to sialic acid as a component of the envelope using a neuraminidase derived from Gram-positive bacteria. More specifically, it relates to:
(1) a method for producing a viral vector comprising a membrane protein that binds to sialic acid, comprising the steps of culturing cells producing the viral vector in the presence of a neuraminidase derived from a Gram-positive bacterium, and recovering the produced virus;
(2) the method of (1), wherein said Gram-positive bacterium is an actinomycete;
(3) the method of (2), wherein said actinomycete belongs to the Micromonosporaceae family;
(4) the method of (3), wherein said actinomycete belonging to the Micromonosporaceae family is *Micromonospora viridifaciens*;
(5) the method according to any one of (1) to (4), wherein said viral vector is a retroviral vector;
(6) the method of (5), wherein said retroviral vector is a lentiviral vector;
(7) the method according to any one of (1) to (6), wherein said membrane protein that binds to sialic acid is an envelope protein of a single stranded negative strand RNA virus;
(8) the method of (7), wherein said single stranded negative strand RNA virus is a virus belonging to the Paramyxoviridae or Orthomyxoviridae family;
(9) the method according to any one of (1) to (6), wherein said membrane protein that binds to sialic acid is an HA protein of an influenza virus; and
(10) a viral vector produced using the method according to any one of (1) to (9).

This invention provides methods of producing a viral vector containing a membrane protein that binds to sialic acid as a component of the envelope. Herein, "viral vector" means a virus particle capable of introducing a nucleic acid molecule into a host or infectious microparticle equivalent to it. The method of this invention comprises the steps of culturing viral vector-producing cells in the presence of neuraminidase derived from a Gram-positive bacterium, and collecting the produced virus. The inclusion of a neuraminidase from a Gram-positive bacterium significantly increases the amount of virus collected from the virus-producing cells. Neuraminidase derived from *Actinomycetes* is preferably used. The method of this invention can be applied for producing a desired viral vector containing an envelope derived from the plasma membrane. For example, the method of this invention may be preferably used to produce a negative strand RNA virus, retrovirus, poxvirus, herpes virus, and the like. In particular, the method of this invention is suitable for production of a retrovirus including lentivirus.

Any replication competent viruses or replication deficient viruses may be produced. For example, replication deficient viruses may be prepared by deleting from the viral genome a part or all of the virus gene(s) participating in the formation of infectious viral particles or their release. Specifically, a recombinant viral vector that is replication deficient, suitable for introducing a desired gene into the cell, can be produced by altering the viral genome so that the gene encoding the envelope or such is deleted while the nucleotide sequence(s) required for the incorporation of the viral genome into viral particle and the introduction of the viral nucleic acid(s) into the target cell are kept intact. For example, a replication deficient retrovirus may be created by removing a part or all of a gene encoding a viral protein such as gag, pol, and env from the viral genome RNA, while retaining only the sequence essential for virus packaging and gene transfection into the target cell (such as a part of the LTR). For producing viral particles, the genes necessary for their formation, among the deleted genes, may be expressed in virus-producing cells. Such viral particles, comprising a genome in which at least a part of the viral genes of the wild-type virus is deleted, are also included in the viral vectors in this invention.

In particular, the present invention is suitably used for production of recombinant virus. "Recombinant virus" means a virus generated using a recombinant polynucleotide. Recombinant polynucleotide means a polynucleotide in which one or both terminals are not linked in the same manner as in nature. Specifically, a recombinant polynucleotide is a polynucleotide in which the connection of the polynucleotide chain is altered (cut, or combined) artificially. Recombinant polynucleotides can be prepared using the combination of polynucleotide synthesis, treatment with nucleases, treatment with ligase, and such through methods commonly known in the art of recombinant DNA technology. A recombinant protein is a protein generated using a recombinant polynucleotide, or a protein synthesized artificially. For example, recombinant proteins may be produced by expressing a recombinant polynucleotide encoding it. Recombinant virus may be generated by expressing a polynucleotide encoding a viral genome constructed through genetic engineering, and reconstituting the virus.

A viral vector produced using the methods of this invention contains in its viral envelope a protein that binds to sialic acid. Such a viral vector may be a virus containing a protein that binds to sialic acid naturally, or one not containing such protein naturally but rather engineered to artificially contain the protein in the envelope. A virus engineered to contain in its envelope a protein that is not found in the envelope of a natural virus (at all or contained very little) is called a pseudotyped virus regarding the protein. A pseudotyped virus that contains a different envelope protein may be produced, for example, by expressing the protein in virus-producing cells. Such a method is particularly suitable in the context of this invention, to produce a pseudotyped virus containing a membrane protein that binds to sialic acid.

A membrane protein that binds to sialic acid includes a protein containing a sialic acid binding region in the extracellular domain. Such a protein is not limited to any particular one; it may be a natural protein or an artificial protein. Membrane proteins that naturally bind to sialic acid are found in many virus envelope proteins, for example. A protein with such a capability can be identified by the activity of inducing hemagglutination (HA). A virus protein with the HA activity is often called hemagglutinin. Such a viral protein having HA activity is particularly suitable for production of the virus of this invention.

The HA activity is found in a variety of viruses. A protein carrying the HA activity of those viruses may be used for the production of the virus of this invention as it is or through alteration, by making a chimeric protein with another protein(s), or such. The HA activity can be detected by using red blood cells from various species. The kind of red blood cells and optimal temperature for the reaction used frequently for detection of the HA activity are appropriately controlled according to the type of virus. For example, the calcium ion is reported to be essential for the reaction using rubella virus and the like. Likewise, in the case of arboviruses, the optimal pH for the reaction is strict. In enterovirus, rubella virus, and such, a virion itself is a protein carrying the HA activity. In viruses such as arbovirus and adenovirus, a protein carrying the HA activity exists as a particle smaller than a virion as well. Poxvirus hemagglutinin exists as a lipid-containing particle distinct from the virion. These proteins carrying the HA activity can be used to produce a virus having HA activity. Adenoviruses of the subgroup III induce incomplete agglutination, i.e., partial hemagglutination, of rat red blood cells. Such a protein may also be used as a membrane protein carrying the HA activity.

The HA activity of a particular viral protein (HA titer) may be tested by a commonly known method (Kokuritsu Yobou Eisei Kenkyujo Gakuyukai (ed.), General experimental virology, 2nd ed. pp. 214-225. Maruzen). Red blood cells may be prepared from chickens (including young and adult), geese, rats, guinea pigs, rhesus monkeys, green monkeys, or humans, for example. The temperature for incubation may be 0°C, 4°C, room temperature, or 37°C, conditions appropriate for each protein. Examples of conditions for the hemagglutination reaction using different viruses are discussed below.

The HA reaction associated with adenovirus is normally independent of pH, and performed at 37°C, for example. For adenoviruses of subgroup I, such as type 3, 7, 11, 14, 16, 20, 21, 25, and 28, for example, red blood cells from rhesus monkeys may be used. For adenoviruses of subgroup II, such as type 8, 9, 10, 13, 15, 17, 19, 22, 23, 24, 26, and 27, for example, rat red blood cells may be used. For adenoviruses of subgroup III, such as type 1, 2, 4, 5, and 6, rat red blood cells may be used to induce incomplete agglutination.

The HA reaction associated with enterovirus is normally pH-independent. Among such, for Coxsackievirus of type A7, for example, chicken red blood cells may be used, and hemagglutination is induced at room temperature. For Coxsackievirus of type A20, A21, and A24, human red blood cells of type O may be used, for example, and hemagglutination is induced at 4°C. For Coxsackievirus of type B1, B3, and B5, human red blood cells of type O may be used, for example, and hemagglutination is induced at 37°C. For Echovirus of type 3, 6, 7, 11, 12, 13, 19, 20, 21, 24, and 29, for example, human red blood cells of type O may be used, for example, and hemagglutination is induced at 4°C.

The HA reaction associated with reovirus is normally pH-independent, and performed at room temperature. For example, for type 1 and type 2, human type O red blood cells may be used, and for type 3, bovine red blood cells may be used.

The HA reaction associated with negative strand RNA viruses, for example, with influenza virus, is performed at approximately pH 7.2. For type A and type B influenza virus, red blood cells may be prepared from chickens, humans, or guinea pigs, and hemagglutination is induced at room temperature. For type C, chicken red blood cells may be used, and the reaction is performed at 4°C. For mumps virus and Newcastle disease virus (NDV), chicken red blood cells may be used, for example, and the reaction is performed at room temperature at pH 7.2 approximately. For parainfluenza virus, the HA reaction is normally pH independent, and chicken or human red blood cells may be used for type 1, and chicken red blood cells may be used for type 2 virus. The reaction is performed at 4°C, for example. For parainfluenza virus of type 3, the reaction may be performed at 4°C to room temperature using human or guinea pig red blood cells. For measles virus, the reaction may be performed using red blood cells from green monkeys, for example, at 37°C.

With arbovirus, the reaction is carried out strictly under acidic conditions, using red blood cells from geese or chicks, for example, at 37°C. For rhabdovirus, red blood cells from geese may be used. For rabies virus, the reaction may be performed preferably at pH 6.4, at 0°C, for example. For vesicular stomatitis virus (VSV), the reaction may be performed preferably at pH 5.8, at 0°C, for example. For poxviruses including vaccinia virus and variola virus, the reaction is normally independent of pH, and carried out using chicken red blood cells at room temperature to 37°C, for example. For rubella virus, the reaction may be performed using red blood cells from chicks or geese at 4°C, at approximately pH 6.2 or 7.2, for example. For polyoma virus, the reaction may be performed using guinea pig red blood cells at 4°C, pH 7.2, for example. For rat virus (RV), the reaction may be performed using guinea pig red blood cells at room temperature, pH 7.2, for example. Any virus protein carrying the HA activity as described above, or its altered protein may be used for producing virus according to the method of this invention. Here, altered protein means a protein in which one or more amino acids are deleted, substituted, and/or inserted into a natural protein. Such a protein may also be used so long as it is a membrane protein that binds to sialic acid. Altered protein may comprise preferably a part of the amino acid sequence of the original protein, more preferably eight amino acids or more, more preferably nine amino acids or more, more preferably ten amino acids or more, and most preferably 15 amino acids or more of the original protein. Alternatively, the identity of an altered protein at amino acid sequence level compared with the original protein may be 70% or higher, more preferably 80% or higher, more preferably 85% or higher, and most preferably 90% or higher. Altered protein may be the original protein or a part of it to which other protein(s) is attached.

Among the membrane proteins comprised in the envelope of a viral vector that bind to sialic acid, specific examples of proteins particularly favorable for the use in this invention include the HN protein of Paramyxovirus, the HA protein of Orthomyxovirus, the E1 protein of Togavirus, the A27L, H3L, and D8L proteins of vaccinia virus, the M and E proteins of Flavivirus, the E1 and E2 proteins of Coronavirus, and the G 1 protein of Bunyavirus. The envelope proteins of a single stranded negative strand RNA virus are particularly favorable; in particular, the HA protein of Orthomyxovirus is favorable.

"Single stranded negative strand RNA virus" means a virus having a single stranded negative strand ((-) strand) RNA as its genome. Examples include viruses of the Paramyxoviridae family such as *Paramyxovirus, Morbillivirus, Rubulavirus,* and *Pneumovirus,* viruses of the Rhabdoviridae family such as *Vesiculovirus, Lyssavirus,* and *Ephemerovirus,* viruses of the Firoviridae family, viruses of the Orthomyxoviridae family such as influenza virus A, B, C, and thogoto-like viruses, viruses of the Bunyaviridae family such as *Bunyavirus, Hantavirus, Nairovirus,* and *Phlebovirus,* and viruses of the Arenaviridae family. In particular, the HA protein of a virus of the Orthomyxoviridae family is preferably used; the HA protein of influenza virus is preferred. In addition, the HN (or H) protein of the Sendai virus, rabies virus, or measles virus, for example, is also suitable. These proteins may be used in combination of other proteins. The proteins may be derived from a natural strain of virus, the wild type-virus, a mutant strain of the virus, a laboratory cultured strain, artificially constructed strain, and the like. The proteins may be a protein generated by alteration of a natural protein.

The membrane protein that binds to sialic acid may have another activity in addition to the sialic acid binding activity. For example, the HN protein of Paramyxovirus carries neuraminidase (NA) activity in addition to sialic acid binding activity (HA activity). While the NA activity of an HN protein is by itself capable of promoting virus production, it is possible to produce virus more efficiently by using it together with NA from Gram-positive bacteria according to the method of this invention. Furthermore, two or more kinds of membrane proteins that bind to sialic acid may be used in combination in this invention. For example, the HA protein of Orthomyxovirus and the HN protein of Paramyxovirus may be used together to produce a pseudotyped virus of the two proteins in the method of this invention. The NA activity of the HN protein and the NA protein derived from Gram-positive bacteria should enable the release of virus from the cell at higher efficiency.

The methods of this invention result in the production of a viral vector containing a membrane protein that binds to sialic acid by culturing cells producing the viral vector in the presence of neuraminidase derived from Gram-positive bacteria in the steps of production of the viral vector. That is, the virus producing cells are cultured in a culture medium containing neuraminidase from a Gram-positive bacterium. Subsequently, the produced viruses are obtained by the step of recovering the produced viruses from the cell. Virus may be recovered by collecting the culture supernatant of virus producing cells, for example. Furthermore, a step of recovering virus from the culture supernatant may be included. Centrifugation, adsorption, filtration, or so may be performed to further purify or concentrate virus from the culture supernatant.

Neuraminidase (NA) (EC 3.2.1.18) is a protein capable of breaking a glycoside bond in sialic acid (an activity referred to herein as the NA activity). Specifically, NA is a protein capable of breaking a glycoside bond in sialic acid in glycoproteins or glycolipids on the cell membrane (Schauer R. Adv. Carbohydr. Chem. Biochem. 40: 131-234 (1982); Cabezas J.A. Biochem. J. 278: 311-312 (1991)). NA activity is defined by a unit (U), by which one unit is the amount of enzyme required for generation of 1 µmol of N-acetylneuraminic acid (NANA) in 1 minute at 37°C, pH 5.0 using N-acetylneuraminyllactose (NANA-lactose) or bovine submaxillary mucin as a substrate (Cassidy J.T. *et al*. J. Biol. Chem. 240: 3501 (1965)). NA is called sialidase in some cases.

Herein, NA derived from a Gram-positive bacterium refers to NA possessed in the Gram-positive bacterium, or a structural equivalent to it, or an altered form of those. For example, NA derived from a Gram-positive bacterium includes NA that can be obtained from a Gram-positive bacterium. For example, it includes NA purified from a Gram-positive bacterium culture, or its recombinant form. A recombinant form of NA from a Gram-positive bacterium may be produced by expressing a gene encoding NA in the same or different kind of organism. NA derived from a Gram-positive bacterium includes those obtained from other source material(s), so long as they are equivalent to the NA protein obtained from the Gram-positive bacterium. In addition, NA derived from a Gram-positive bacterium includes a protein that is modified, in which one or more amino acids are deleted, substituted, and/or inserted into the wild-type NA from the Gram-positive bacterium, so long as the modified protein retains NA activity,. The number of altered amino acids is not limited so long as the NA activity is retained, but it is preferably 70 amino acids or less, more preferably 50 amino acids or less, more preferably 30 amino acids or less, more preferably 20 amino acids or less, more preferably 10 amino acids or less, more preferably 5 amino acids or less, and most preferably 3 amino acids or less. The identity of altered NA compared to the wild-type NA from a Gram-positive bacterium at amino acid sequence level is 50% or higher, preferably 60% or higher, more preferably 70% or higher, more preferably 80% or higher, more preferably 85% or higher, and most preferably 90% or higher. The identity of amino acid sequences may be determined by BLAST, for example, the algorithm developed by Karlin and Altschul (Karlin S. and Altschul S.F. Proc. Natl. Acad. Sci. USA 87: 2264-2268 (1990); Proc. Natl. Acad. Sci. USA 90: 5873-5877 (1993)). The BLAST program may be run using default parameters. Specific procedures for such analyses are commonly known (refer to the website of BLAST (Basic Local Alignment Search Tool) in NCBI (National Center for Biotechnology Information)). For example, using the blast2sequences program, a program for comparing two sequences, alignment of two sequences may be generated, and their identity may be determined (Tatiana A. *et al*. FEMS Microbiol. Lett. 174: 247-250 (1999)). Occurrence of a gap may be treated in the same way as a mismatch, and the identity, for example, compared to the entire amino acid sequence of the wild-type NA from a Gram-positive bacterium may be calculated. In altering amino acids, it is desirable to alter amino acids in a region other than the BNR (bacterial neuraminidase repeat; Pfam Accession number PF02012 (also called "the Asp box")) so that the BNR, which is important for the NA activity, is not disrupted (Roggentin P. *et al*. Glycoconj. J. 6: 349-353 (1989); Copley R.R. *et al*. Protein Sci. 10: 285-292 (2001)). It is preferable for multiple copies of BNR to exist in a repeated manner with each repeat apart from others by 40 amino acids or further. Alteration of the N- and/or C-terminal amino acids of the wild-type NA is expected to have a minimal effect on the activity, if any.

Herein, NA derived from a Gram-positive bacterium includes a partial protein of the wild type NA from a Gram-positive bacterium, having the NA activity. Such a partial protein comprises preferably 60% or more of the entire amino acid sequence of the wild-type NA of the Gram-positive bacterium in a continuous manner, more preferably 70% or more, more preferably 75% or more, more preferably 80% or more, more preferably 85% or more, and most preferably 90% or more. Furthermore, NA from a Gram-positive bacterium may include a protein having the NA activity, in which other protein(s) is attached to the wild-type NA of the Gram-positive bacterium, or its partial protein. Preparation of a partial protein or a fusion protein with other protein(s) while keeping the activity is a common practice to one skilled in the art.

Gram-positive bacterium means a bacterium or other kind of fungus that is positive by the Gram staining. Being positive by the Gram staining means generally that the bacterium is resistant to discoloring after staining in the basic staining solution (Crystal violet, for example), followed by treatment with the Lugol's solution (I₂-KI solution), and then by a brief wash with a polar solvent (alcohol, acetone, or so). By counter-staining (with safranin solution or dilute carbol-fuchsin solution, for example) after washing, Gram-negative bacteria turn red, whereas Gram-positive bacteria turn purple. Generally, Gram-positive bacteria have a relatively thick cell wall (15 to 80 nm), and many of them lack lipopolysaccharide in the outer layer. In addition, many of them are highly sensitive to lysozyme. Specific examples of Gram-positive bacteria include *Staphylococcus, Streptococcus, Bacillus subtilis, Bacillus megaterium,* and actinomycetes. In the present invention, NA derived from a Gram-positive bacterium is preferably NA from actinomycetes.

Actinomycetes mean bacteria of the order Actinomycetales, and other Actinobacteria. Actinomycetes belong to a group of Gram-positive bacteria. The order Actinomycetales includes the taxonomic families of Frankiaceae, Micromonosporaceae, Propionibacteriaceae, Pseudonocardiaceae, Streptomycetaceae, Streptosporangiaceae, Thermomoriosporaceae, Corynebacteriaceae, Mycobacteriuaceae, Nocardioidaceae, Actinomycetaceae, Micrococcaceae, Actinosynnemataceae, Nocardiopsaceae, and Glycomycetaceae. For example, reference to NA *of Actinomyces* is found in the Accession Number L06898 (protein: AAA21932, A49227), and X62276 (protein: CAA44166) (NA of *Actinomyces viscosus*). Reference to NA of *Corynebacterium* is found in the Accession Number NC_003450 (protein: NP_600771), and AP005278 (protein: BAB98949) (NA of *Corynebacterium glutamicum),* for example. Reference to NA of *Streptomyces* is, for example, the Accession Number NC_003155 (protein: NP_823018) (*Streptomyces avermitilis*), and NC_003888 (protein: NP_630638) *(Streptomyces coelicolor*).

The neuraminidase used herein is preferably derived from a bacterium of the order Actinomycetales, more preferably from the family Micromonosporaceae, and most preferably from the genus *Micromonospora.* Bacteria of *Micromonospora* include *M. aurantiaca, M. brunnea, M. brunnescens, M. carbonacea, M. cellulolyticum, M. chalcea, M. chersinia, M. citrea, M. coerulea, M. echinoaurantiaca, M. echinobrunnea, M. echinospora, M. floridensis, M. fulviviridis, M. fulvopurpureus, M. fulvoviolaceus, M. globosa, M. griseorubida, M. halophytica, M. inositola, M. inyoensis, M. lacustris, M. megalomicea, M. melanospora, M. narashino, M. olivasterospora, M. peucetica, M. purpurea, M. purpureochromogenes, M. rhodorangea, M. rosaria, M. rosea, M. sagamiensis, M. viridifaciens, M. yulongensis,* and *M. zionensis,* but may not be limited thereto. A particularly favorable NA is NA from *M. viridifaciens* (ATCC 31146). The nucleotide sequence of the *M. viridifaciens* NA gene (*nedA*) is described in the Accession number D01045 (SEQ ID NO: 1), and the amino acid sequence of encoded protein is described in Q02834 (SEQ ID NO: 2) (Sakurada K. *et al*. J. Bacteriol. 174(21): 6896-6903 (1992)).

In addition, the NA gene of another *Micromonospora* bacterium may be isolated using the NA gene of *M. viridifaciens* as a probe. For example, a nucleic acid that hybridizes with the nucleotide sequence of SEQ ID NO: 1 and/or its complementary sequence of 30 nucleotides or longer, more preferably 50 nucleotides or longer, more preferably 100 nucleotides or longer, more preferably 150 nucleotides or longer, and most preferably its entire sequence under stringent conditions may be selected. Such nucleic acid may be identified by preparing a probe from either nucleic acid comprising the nucleotide sequence of SEQ ID NO: 1, or the target nucleic acid in hybridization, and detecting hybridization of the probe to the other nucleic acid. Stringent conditions may be achieved by performing hybridization in a solution containing 5x SSC (1x SSC: 150 mM NaCl and 15 mM sodium citrate), 7% (w/v) SDS (sodium dodecyl sulfate), 100 µg/ml of denatured salmon sperm DNA, and 5x Denhardt's solution (1x Denhardt's: 0.2% polyvinylpyrrolidone, 0.2% bovine serum albumin, and 0.2% Ficoll) at 48°C, preferably at 50°C, more preferably at 55°C, and most preferably at 60°C, followed by washing with agitation in 2x SSC, 0.1% (w/v) SDS for 2 hours at the same temperature used for hybridization. More preferably, washing may be carried out for 2 hours with agitation in 1x SSC, 0.1% (w/v) SDS, more preferably in 0.5x SSC, 0.1% (w/v) SDS, and most preferably in 0.1x SSC, 0.1% (w/v) SDS.

In the method of producing virus of the present invention, virus producing cells are cultured in the presence ofNA derived from Gram-positive bacteria as described above, and the produced virus is collected from the culture. As the virus is released from the cells into the culture medium, it can be recovered by collecting the culture supernatant. In producing a pseudotyped virus of a sialic acid binding protein, cells expressing the protein are cultured in the presence of NA from a Gram-positive bacterium for virus production, and the produced virus is recovered. NA from a Gram-positive bacterium may be supplied by its addition to the culture medium, or may be secreted from virus producing cells, or other cells co-cultured with the virus producing cells, using an expression vector of the NA. NA must be present for at least a portion of the time during which the virus producing cells are cultured. The duration in which the virus producing cells are cultured in the presence of NA may be 1 hour or longer, for example, preferably 3 hours or longer, more preferably 5 hours or longer, more preferably 10 hours or longer, and most preferably 20 hours or longer. Preferably, the virus producing cells are cultured in the presence of NA from a Gram-positive bacterium for 1 hour or longer before recovering the produced virus from the culture, more preferably 3 hours or longer, more preferably 5 hours or longer, more preferably 10 hours or longer, and most preferably 20 hours or longer. During the time, viruses accumulate in the culture supernatant.

The amount of neuraminidase from a Gram-positive bacterium in the culture may be adjusted appropriately for the maximal virus production. The concentration of NA from a Gram-positive bacterium in the culture medium is, for example, 0.1 mU/ml to 1000 U/ml, preferably 0.2 mU/ml to 500 U/ml, more preferably 0.5 mU/ml to 100 U/ml, and most preferably 1 mU/ml to 50 U/ml. In particular, NA from a Gram-positive bacterium is excellent because it is capable of producing a virus having a sufficient titer even at 10 U/ml or lower. For example, it is capable of releasing high titer virus from virus producing cells at a low concentration, such as 1 U/ml or lower, 0.1 U/ml or lower, 0.05 U/ml or lower, or 0.01 U/ml or lower.

A viral vector produced using the method of this invention may contain another protein in the envelope, in addition to a sialic acid binding protein as described above. For example, in addition to an envelope protein having the HA activity, such as an HA (or HN) protein of a negative strand RNA virus, another envelope protein participating in membrane fusion such as an F protein of a negative strand RNA virus may be further included. Moreover, an envelope protein derived from a desired virus may be further included. For example, an envelope protein derived from a virus capable of infecting human cells may be suitably used. Such a protein is not limited to any specific one; it includes an amphotropic envelope protein of retrovirus, and a G protein of vesicular stomatitis virus (VSV). In addition, it includes the herpes virus proteins such as the gB, gD, gH, and gp85 proteins of the Herpes Simplex virus, and the gp350 and gp220 proteins of the EB virus, for example. It also includes Hepadnavirus proteins, such as the S protein of the Hepatitis B virus.

For example, a vector comprising a retroviral amphotropic envelope protein (amphotropic env) and an HA (or HN) protein of a negative strand RNA virus may be produced according to the method of this invention. In addition, a virus produced by the method of the invention may comprise the VSV-G protein, a glycoprotein on the surface of vesicular stomatitis virus (VSV), for example. VSV-G is thought to bind to phospholipids present in most animal cells as the receptor. Thus, the use of a vector comprising a VSV-G protein and a sialic acid binding protein dramatically increases the types of cells amenable to gene transfection, and improves transfection efficiency as well (WO01/92508). An example of such is a vector containing a VSV-G protein and an HA (or HN) protein of a negative stand RNA virus. Such vectors may further contain an F protein of a negative strand RNA virus. Thus, the present invention is useful for production of a vector containing a retroviral amphotropic envelope protein, an F protein, and an HA (or HN) protein, and a vector containing a VSV-G protein, an F protein, and an HA (or HN) protein. Furthermore, such vectors may contain an M protein of a negative strand RNA virus. Thus, the present invention is suitably used for production of a vector containing a retroviral amphotropic envelope protein, an F protein, an HA (or HN) protein, and an M protein, and a vector containing a VSV-G protein, an F protein, an HA (or HN) protein, and an M protein. Such a vector as described above is excellent in providing a highly efficient gene transfection into cells that have been difficult to introduce genes by conventional methods, such as mucous cells, cellular fractions containing hematopoietic stem cells, and the like. In addition, because the VSV-G protein is present on the membrane as a stable trimer formed by a single glycoprotein, such a vector particle is less prone to disruption during purification steps, and thus, it is possible to concentrate it highly by centrifugation (Yang Y. *et al*. Hum. Gene Ther. 6(9): 1203-1213 (Sep. 1995)).

The HA (or HN), F, and M proteins of a negative strand RNA virus used for producing a pseudotyped virus are not limited to any specific ones. In particular, proteins of a virus of Orthomyxoviridae, and Paramyxoviridae may be suitably used. Specifically, for example, the envelope proteins of influenza virus and Sendai virus may be suitably used. For example, a pseudotyped virus produced using an HA protein of an influenza virus expressed in virus producing cells will be capable of infecting a broad range of mammalian cells including human cells. The HA (or HN), F, and M proteins of a negative strand RNA virus and such may be prepared from a desired virus strain. For example, proteins of a pathogenic virus may be derived from an attenuated strain or boosted strain. For example, the HN, F, and M proteins of Sendai virus may be derived from the Z strain. Similarly, the envelope proteins of influenza virus may be derived from a desired isolated strain.

In addition, a retroviral amphotropic envelope protein may be derived from Murine leukemia virus (MuLV) 4070A strain. The envelope proteins of MuLV 10A1 strain may be used as well (for example, pCL-10A1 (Imgenex)) (Naviaux R.K. *et al*. J. Virol. 70: 5701-5705 (1996)). Ecotropic envelope proteins may be derived from Moloney murine leukemia virus (MoMuLV). VSV-G protein may be derived from the Indiana serotype strain (J. Virol. 39: 519-528 (1981)), for example. Furthermore, proteins derived from any desired strain may be used as well.

The above envelope proteins, such as the HA (or HN), F, G, and M proteins of a negative strand RNA virus or retroviral envelope proteins, may comprise intact proteins expressed in the wild-type virus, or may contain a naturally-occurring or artificially introduced mutation(s). For example, it is possible to analyze envelope proteins for epitopes that might become cell surface antigen molecules. Then, using the information, a protein with attenuated antigenicity may be selected for virus production.

For example, it is possible to produce a vector having higher gene transfection efficiency by making a pseudotyped viral vector using a protein in which the cytoplasmic domain of a viral envelope protein that binds to sialic acid, such as HA or HN protein, or other envelope protein(s) is altered by deletion, substitution, and/or addition. Thus, the present invention relates to a method of producing a pseudotyped viral vector comprising a protein in which a part or the entire region of the cytoplasmic domain of a naturally occurring membrane protein that binds to sialic acid is altered by deletion, substitution, and/or addition. Specifically, an altered protein, such as those in which the cytoplasmic region of the HA (or HN) and/or F proteins of a negative strand RNA virus is deleted, or substituted or attached by the cytoplasmic region of other membrane proteins (for example, envelope proteins of retroviruses including Lentivirus), may be suitably used for production of a viral vector having high infectivity. Particularly, a protein in which the cytoplasmic region of a viral envelope protein that binds to sialic acid is altered by substitution, deletion, and/or addition to the wild-type protein may be used for production of a pseudotype viral vector with increased infectivity. For example, substitution or attachment of the cytoplasmic region of a retroviral envelope protein may be suitable for producing a retroviral vector.

Specifically, by making a pseudotyped retrovirus using a protein in which the cytoplasmic region of an HA (or HN) protein of a negative strand RNA virus is substituted with that of an envelope protein of a retrovirus or Lentivirus such as SIV, and a protein in which HA (or HN) protein of a negative strand RNA virus is attached with the cytoplasmic region of an envelope protein of a retrovirus such as Lentivirus, it is possible to transfer a foreign gene at high efficiency into a broad range of cells including human cells. The length of the deleted cytoplasmic region, and the length of the attached cytoplasmic region of a retroviral envelope protein, are not limited in particular; all or part of the cytoplasmic region may be deleted, substituted, and/or attached.

Such viral vectors as above may further comprise an altered F protein of a negative strand RNA virus. For example, a protein in which the cytoplasmic region of an F protein of a negative strand RNA virus is deleted, and a protein in which the above deleted F protein is attached with the cytoplasmic region of an envelope protein of a retrovirus or lentivirus, such as SIV, may be used. Specifically, a plasmid may be constructed for expressing a protein in which amino acids in the cytoplasmic region of the F protein are deleted, for example. The length of the deletion is not limited in particular; all or part of the cytoplasmic region may be deleted. For example, the F protein in which the cytoplasmic region is deleted, leaving zero to a few amino acids, is considered suitable for producing a pseudotyped retrovirus. Such a deleted F protein may be attached to all or part of the cytoplasmic region of an envelope protein of other virus (Lentivirus envelope protein, for example), and used for virus production as a protein in which the cytoplasmic region of F protein is substituted with other peptide. An example of such proteins may be a protein attached with 11 amino acids from the 5'-side of the cytoplasmic region of SIV envelope protein. Thus, the present invention also relates to a method of producing a pseudotyped virus, further using an F protein of a negative strand RNA virus, in which all or part of the naturally-occurring cytoplasmic region of the protein is altered by substitution, deletion, and/or attachment. In particular, the present invention further relates to a method of producing a pseudotyped virus, comprising further a protein in which the cytoplasmic region of the F protein is substituted with a part or the entire of the cytoplasmic region of an envelope protein of a retrovirus including Lentivirus.

Furthermore, the extracellular domain of a viral envelope protein may be substituted with a membrane protein having the cell adhering activity, a protein such as adhesion molecule, ligand, or receptor, or antibody or its fragment to create a chimeric protein. Such chimeric proteins may be used for producing a vector capable of infecting a broad range of tissues or a specific tissue as its target.

The method of the present invention is particularly suited for production of a retroviral vector. A retrovirus is a virus having a genome of a (+) sense strand RNA, containing reverse transcriptase characteristically. It converts its RNA genome into DNA using reverse transcriptase upon infecting a target cell, and integrates into the chromosomes of the target cell. Such viruses are collectively called "retroviruses". Herein, the retrovirus may be an altered form of the wild-type retrovirus. Such an altered form of virus should comprise at least a part of the retroviral genome RNA in the RNA contained in the viral particles, and exist as an infectious viral particle generated by the incorporation of the RNA in a sequence dependent manner through the action of retroviral proteins during viral particle formation. More specifically, the genome RNA of a retrovirus preferably comprises a packaging signal sequence essential for the incorporation into viral particles. Transcription of an RNA having an LTR on each end, comprising such signal sequence, in the presence of retroviral proteins leads to the formation of viral particles containing the RNA.

Herein, retroviral vectors include those derived from oncovirus. The term "oncovirus" refers to a retrovirus belonging to the subfamily Oncovirus. Oncoviruses include retroviruses related to tumorigenesis, such as sarcoma virus, leukemia virus, and mammary tumor virus. For example, Moloney murine leukemia virus (MoMLV) is one of the earliest developed retroviral vectors, which has been modified by many improvements, and widely used. The method of this invention may be suitably used for producing a pseudotype viral vector of MoMLV using a sialic acid binding protein such as HA (or HN) protein of a negative strand RNA virus. In addition, Murine stem cell virus (MSCV) is suitably used as a vector for gene transfer into blood cells, hematopoietic cells, and embryonic stem cells, in particular. For example, using a pseudotype MSCV prepared with an envelope protein of a negative strand RNA virus, having the HA activity, it is possible to introduce a gene into CD34 positive cells from bone marrow including hematopoietic stem cells at high efficiency.

Herein, the retroviral vector also includes those derived from lentivirus. The term "lentivirus" refers to a retrovirus belonging to the lentivirus subfamily (Lentivirus). Examples of lentiviruses include human immunodeficiency virus (HIV) *(e.g.,* HIV1 or HIV2), simian immunodeficiency virus (SIV), feline immunodeficiency virus (FIV), Maedi-Visna virus, equine infectious anemia virus (EIAV), caprine arthritis-encephalitis virus (CAEV), etc. The retroviral vector can be derived from a desired strain or subtype. For example, HIV-1 includes those of every major (M) subtype (including A to J), N, and outlier (O) (Hu, D. J. *et al*., JAMA 1996; 275: 210-216; Zhu, T. *et al*., Nature 1998, 5; 391(6667): 594-7; Simon, F. *et al*., Nat. Med. 1998, 4(9): 1032-7). Isolated SIV strains include, for example, SIVagm, SIVcpz, SIVmac, SIVmnd, SIVsnm, SIVsyk, etc.

An advantage of lentiviruses is that they are infectious to nondividing cells and the viral genome can be integrated into host cell chromosome. The nuclear translocation signals and integrases encoded by gag and vpr are believed to be responsible for the integration. When, using this characteristic, a viral vector is constructed based on a lentivirus according to the present invention, genes can be introduced into nondividing cells in living tissues and cells that hardly divide, such as stem cells in various tissues, which allows for the efficient production of vectors capable of long-term gene expression.

Human immunodeficiency virus (HIV), before any other lentivirus, was used to construct a vector, which can also be used preferably in the method of the present invention. In addition, vectors have been developed based on simian immunodeficiency virus (SIV), feline immunodeficiency virus (FIV) (Poeschla, E. M. *et al*., Nature Medicine, 4(3), 354-7, 1998), caprine arthritis-encephalitis virus (CAEV) (Mselli-Lakhal, L. *et al*., Arch. Virol., 143(4), 681-95, 1998), equine infectious anemia virus (EIAV), and bovine immunodeficiency virus (BIV). The method of the present invention can be applied to the production of these vectors.

Simian immunodeficiency virus (SIV) was discovered as a monkey-derived HIV-like virus, which, along with HIV, forms the group of primate lentivirus (E. Ido and M. Hayamizu, "Gene, Infection and Pathogenicity of Simian Immunodeficiency Virus", Protein, Nucleic acid and Enzyme, Vol.39, No.8, 1994). This group is further divided roughly into four subgroups: (1) HIV-1 subgroup containing HIV-1 that is the causative virus for human acquired immune deficiency syndrome (AIDS) and SIVcpz isolated from chimpanzee; (2) HIV-2 subgroup containing SIVsmm isolated from Sooty Mangabey *(Cercocebus atys*), SIVmac isolated from rhesus monkey (*Macaca mulatta*), and HIV-2 that is less pathogenic in human (Jaffar, S. *et al*., J. Acquir. Immune Defic. Syndr. Hum. Retrovirol., 16(5), 327-32, 1997); (3) SIVagm subgroup represented by SIVagm isolated from African green monkey (*Cercopithecus aethiops*); and (4) SIVmnd subgroup represented by SIVmnd isolated from Mandrill *(Papio sphinx).*

There is no report suggesting the pathogenicity of SIVagm and SIVmnd in natural hosts (Ohta, Y. *et al*., Int. J. Cancer, 15,41(1), 115-22, 1988; Miura, T. *et al*., J. Med. Primatol., 18(3-4), 255-9,1989; M. Hayamizu, Nippon Rinsho, 47, 1, 1989). In particular, previous reports describe that, according to the results of infection experiments, the TYO-1 strain, which is one of the SIVagm subgroup, and was also used herein in the Examples, is not pathogenic in crab-eating monkey (*Macaca facicularis*), rhesus monkey (*Macaca mulatta*) or in other natural hosts (Ali, M. *et al*., Gene Therapy, 1(6), 367-84, 1994; Honjo, S *et al*., J. Med. Primatol., 19(1), 9-20, 1990). There is no report of SIVagm infection to humans and the onset thereof, and thus it is believed that SIVagm may not be pathogenic to human. In general, lentiviruses in primates have strict species-specificity, and there are few reports of cases of cross-species infection with SIVagm from natural hosts and onset thereof; if any, normally the onset frequency is low and the disease progresses slowly (Novembre, F. J. *et al*., J. Virol., 71(5), 4086-91, 1997). Accordingly, a viral vector prepared based on SIVagm, particularly SIVagm TYO-1, is thought to be safer than vectors based on HIV-1 or other lentiviruses, and thus it is a preferred virus produced in the present invention.

Furthermore, in the present invention, the retroviral vector includes those derived from spumavirus. The spumavirus includes, for example, foamyvirus (DE4318387; WO9607749; Virology (1995) 210, 1, 167-178; J. Virol. (1996) 70, 1, 217-22). Vectors derived from foamyvirus can be utilized for introducing foreign genes into human cells, particularly in gene therapy and administration of recombinant vaccines.

In the present invention, the retroviral vector may be modified in the LTR (long terminal repeat). The LTR is a retrovirus-specific sequence which is present in the viral genome at both ends. The 5' LTR serves as a promoter, which enhances the mRNA transcription from the provirus. Thus, a substitution of the portion exhibiting the 5' LTR promoter activity in the vector (herein referred to as gene transfer vector) that expresses RNA to be incorporated into viral vectors with another promoter having stronger promoter activity can lead to increased levels of mRNA transcription of the gene transfer vector, which may improve the packaging efficiency and thus increase the vector titer. Furthermore, for example, in the case of lentivirus, the transcription activity of 5' LTR is known to be enhanced by viral tat protein, and, therefore, substitution of the tat protein-independent promoter for the 5' LTR allows for the exclusion of tat from the vector that expresses viral genes required for viral packaging (herein referred to as packaging vector). The intracellular RNA of virus infected to cells is reverse transcribed and forms a closed circular structure with a linkage between the LTRs at the two ends, and then integrated into the chromosome of cells through the interaction between the linkage site and viral integrase. The mRNA transcribed from the provirus corresponds to the region from the transcription initiation site in the 5' LTR to the 3' LTR polyA sequence that located downstream; the 5' LTR promoter portion is not packaged in the viral particle. Thus, even if the promoter is replaced with another sequence, the portion that is integrated into the chromosome of target cells has no alteration. Based on the facts described above, a substitution of 5' LTR promoter can provide a safer vector with a higher titer. Thus, a substitution of the promoter at the 5' end in a gene transfer vector can increase the titer of packagable vectors.

The safety can be improved with a self inactivating vector (SIN vector) which is prepared by partially eliminating the 3' LTR sequence to prevent the transcription of full-length vector mRNA in target cells thereof. The provirus for lentivirus, which is integrated into the chromosome of target cells, has the U3 portion of 3' LTR attached to the 5' end thereof. Thus, in the chromosome of target cells, the gene transfer vector contains U3 at the 5' end and accordingly the transcription of RNA covering the whole gene transfer vector starts there. If there were lentivirus or related proteins in the target cells, the gene transfer vector would be re-packaged and infect other cells. Furthermore, there is a possibility that a host gene located adjacent to the 3' end of viral genome may be expressed by the 3' LTR promoter (Rosenberg, N., Jolicoeur, P., Retroviral Pathogenesis. Retroviruses. Cold Spring Harbor Laboratory Press, 475-585, 1997). Such events are recognized as being problematic with retroviral vectors. Thus, the SIN vector was developed as a solution to overcome the problems (Yu, S. F. *et al*., Proc. Natl. Acad. Sci. U S A, 83(10), 3194-8, 1986). When the U3 portion is deleted from the 3' LTR in the gene transfer vector, neither 5' LTR nor 3' LTR promoter is present in the target cells. In such cases, neither full-length viral RNA nor host gene is transcribed, while the transcription of genes of interest is achieved with internal promoters; such a vector can be an overexpression vector with higher safety. Such vectors can also be produced according to the method of this invention. The gene transfer vectors for producing SIN vectors can be constructed according to any of methods known in the art (WO01/92508).

Retroviruses can be produced by transcribing a gene transfer vector DNA containing the packaging signal in host cells, and allowing viral particles formation in the presence of gag and pol proteins, and envelope proteins. The gene transfer vector DNA may be a DNA vector such as plasmid, or a DNA that has been integrated in the chromosome of packaging cells. While it is preferable to integrate the packaging signal sequence encoded by the gene transfer vector DNA so long as possible to maintain the structure formed based on the sequence, it is required to minimize the sequence homologous between the packaging signal in the vector DNA and another packaging vector for providing gag and pol proteins to reduce the frequency of wild-type virus formation due to recombination between the types of vectors. Accordingly, it is preferable to construct the gene transfer vector DNA using as short a sequence as possible comprising the sequence required for packaging to meet both criteria of packaging efficiency and safety.

There is no limitation on the type of packaging signal, so long as packaging is achieved in cells where the packaging vector has been introduced; those derived from retrovirus, lentivirus, immunodeficiency virus, and the like can be used depending on the type of packaging vector.

For example, in the case of SIVagm-derived packaging vector, the type of virus from which the signal sequence to be used is derived is limited to SIV because HIV vectors are not packaged. However, the SIV-derived gene transfer vector is also packagable when an HIV-derived packaging vector is used. Thus, the frequency of recombinant virus formation can be reduced when the vector particles are formed by combining gene transfer vector and packaging vector, each derived from different type of lentivirus. In such cases, it is preferred to use combinations of lentiviruses in primates (for example, HIV and SIV).

In a preferred gene transfer vector DNA, gag protein has been modified so that it is not expressed. The viral gag protein can be detected as a foreign substance in the living body, and thus a potential antigen. Alternatively, the protein may affect cellular functions. To prevent the gag protein expression, frame shift mutations can be introduced for modification by adding or deleting nucleotides downstream of the start codon of gag. It is also preferable to delete portions of the coding region of gag protein. In general, a 5' portion of the coding region of the gag protein is known to be essential for viral packaging. Thus, in a gene transfer vector, it is preferred that the coding region for the gag protein be deleted at the C terminus. It is preferred to delete as large a portion of gag coding region as possible, so long as the deletion does not considerably affect the packaging efficiency. In addition, it is preferred to replace the start codon (ATG) of gag protein with a codon other than ATG. Such a codon for the replacement can be selected appropriately not to greatly affect the packaging efficiency. A viral vector containing the transcription product of gene transfer vector DNA can be produced by introducing the constructed gene transfer vector DNA comprising the packaging signal into appropriate packaging cells. The viral vector particles produced can be recovered from the culture supernatant of packaging cells, or the like.

There is no limitation on the type of packaging cell to be used, so long as the cell line is generally used in viral production. When used for the purposes of gene therapy in human, a human or monkey-derived cell is preferred. Human cell lines to be used as packaging cells include, for example, 293 cell, 293T cell, 293EBNA cell, SW480 cell, u87MG cell, HOS cell, C8166 cell, MT-4 cell, Molt-4 cell, HeLa cell, HT1080 cell, TE671 cell, etc. Monkey cell lines include, for example, COS1 cell, COS7 cell, CV-1 cell, BMT10 cell, etc. In addition, previously established packaging cells can be used. Such packaging cells include, for example, Bosc23 cell, PE501 cell, etc.

There is no limitation on the type of foreign gene to be inserted in the vector, which include, for example, nucleic acids which do not encode any protein, such as antisense or ribozyme sequences, as well as protein-encoding nucleic acids.

In recent years, attention has been given to a variety of stem cells, including hematopoietic stem cells, as targets of gene therapy (Y. Hanazono, Molecular Medicine, Vol. 36, No. 7, 1999). A pseudotyped retroviral vector comprising an envelope protein having the hemagglutinin activity of a negative strand RNA virus can transfer genes into CD34-positive cells derived from human bone marrow with high efficiency (WO01/92508); such a fraction comprising the CD34-positive cells receives attention as a cell fraction containing hematopoietic stem cells in recent years. Previous reports describe that the CD34-positive cells exhibit pluripotency in colony assay using a culture medium containing methylcellulose (Kirshenbaum, A. S. *et al*., J. Immunol., 148(3), 772-7, 1992) and that transplantation of CD34-positive cells into NOD/SCID mouse that is a compounded immunodeficiency strain leads to localization of the cells in the mouse bone marrow and reconstitution of hemopoietic system (Larochelle, A. *et al*., Nat. Med., 2(12), 1329-37, 1996). Hence, it is thought that stem cell-like immature cells are present in at least the CD34-positive cell fraction. The hematopoietic stem cells in the CD34-positive cell fraction are in nondividing state. In general, when a retroviral vector is used, the efficiency of gene transfer into such cells is low (Kiem, H. P. *et al*., Curr. Opin. Oncol., 7(2), 107-14, 1995.); however, the infection efficiency can be greatly improved by using a pseudotyped vector comprising an envelope protein having the hemagglutinin activity of a negative strand RNA virus. In particular, the efficiency of gene transfer into nondividing cells is expected to further increase through the use of a lentiviral vector, such as HIV or SIV vector. The method of the present invention for producing a pseudotyped retroviral vector using a protein that binds to sialic acid is useful for production of a vector for gene transfer into blood cells or hematopoietic cells. Thus, the present invention relates to a method for producing a vector for gene transfer into blood cells or hematopoietic cells using the method of the invention for virus production. It also relates to a method of gene transfer into blood cells or hematopoietic cells, comprising a step of contacting the blood cells or hematopoietic cells with a vector produced using the method of this invention, and use of the vector produced by the method for gene transfer into blood cells or hematopoietic cells. The efficiency of transfer of a foreign gene into blood cells or hematopoietic cells may be evaluated, for example, by flow cytometry analysis using antibodies against a variety of known cell surface antigens, colony assay, and reconstitution of the hematopoietic system by transplanting hematopoietic cells into mice whose hematopoietic system is disrupted.

The retroviral vector pseudotyped by the use of proteins having sialic acid binding activity, can transfer genes into hemocytes and hematopoietic cells with high efficiency, and thus is useful in gene therapy whose target is blood cells, *e*.*g*., adenosine deaminase (ADA) deficiency (Blaese, R. M., Pediatr. Res., 33 (1 Suppl), S49-53, 1993), hemophilia (Kay, M. A. *et al*., Proc. Natl. Acad. Sci. USA, 96(18), 9973-5, 1999) and Fanconi anemia, etc. The administration can be performed, for example, by an *ex vivo* method.

Specifically, examples of gene therapies that targets hemopoietic cells, to which the vector produced by the method of the present invention is applicable, include, for example, use of the drug resistance gene MDR1 to preserve stem cells in anti-cancer chemotherapy (Licht, T. *et al*., Gene Ther. (2000) 7, 4, 348-58); introduction of the normal FANCC gene for the treatment of Fanconi anemia (Liu, J. M. *et al*., Hum. Gene Ther. (1999) 10,14, 2337-46); introduction of a combination of cytokines (thrombopoietin, interleukins 6 and 11, and Flt-3 ligand) to enhance the *ex vivo* proliferation of stem cells (WO 99/07831); the expression of chimeric proteins, such as Flt-3 agonist, to treat cytopenia (WO 98/46750); introduction of the human β globin gene to treat β thalassemia (WO 9741141); a combination therapy with IL-6 antagonist and suicide gene expression to treat IL-6-dependent multiple myeloma (German Patent No. DE19704979); introduction of genes such as receptor agonist comprising a combination of hemopoietic factors [interleukins (GM-CSF, G-CSF-Ser17, M-CSF, erythropoietin, IL-1, IL-14, IL-2, IL-5, IL-6, IL-7, IL-8, IL-9, IL-10, IL-11, IL-12, IL-13, and IL-15), leukemia inhibitory factor (LIF), flt3/flk2 ligand, human somatotropin, B cell growth factor, B cell differentiation factor, erythrocyte differentiation factor (EDF) or stem cell factor (SCF)] (WO 97/12985), c-mpl receptor agonist to be used in stem cell culture and gene therapy for hemopoietic diseases (WO 97/12978), IL-6 and IL-6 soluble receptor fusion protein to be used for the proliferation of human hemopoietic progenitor cells (Nat. Biotechnol. (1997) 15, 2, 142-45), IL-6 superagonist and superantagonist to be used to proliferate hemopoietic progenitor cells (WO 96/18648), Factor-X to be used in therapy for blood diseases (J. Cell. Bioche. (1995) Suppl. 21A, 410), stem cell factor, IL-6, and soluble IL-6 receptor complex to be used to proliferate human hemopoietic progenitor cells (Gene Ther. (1995) 2, 9, 694), ribozyme whose targets are RNA viruses, and antisense and/or decoy RNA which are useful to prevent HIV infection and for intracellular immunity (WO 96/22368). This invention relates to methods for producing a viral vector encoding any of the above or any combination thereof, wherein the viral vector comprises a cyalic acid-binding membrane protein in its envelope.

In addition, a vector produced by the method of the present invention has a strong infectivity in mucous cells, such as nasal mucoepithelial cells and lung bronchial mucoepithelial cells. Using conventional viral vectors, it is very difficult to transfer genes into mucous cells such as those in the tracheal epithelium; besides, treatment for removing physical barriers is essential. For example, gene transfer using an HIV vector of VSV-G pseudotype does not give sufficient transfection efficiency unless tissues are damaged by sulfur dioxide, or such (Johnson L.G. *et al*. Gene Therapy 7: 568-574 (2000)). However, vectors produced by the method of this invention are capable of transferring genes into the mucous cells, which have been difficult to transfect, at high efficiency without damaging cells or tissues (WO01/92508). Accordingly, the present invention relates to a method of producing a vector for gene transfer into mucous cells using the method of virus production of this invention. It also relates to a method of gene transfer into mucous cells, comprising a step of contacting mucous cells with a vector produced by the method of this invention, and using the vector produced by the method for gene transfer into mucous cells. Mucous cells include mucous epithelial cells, in particular, and nasal or lung bronchial mucous epithelial cells, specifically.

Specific examples of applications include induction of the immune system by transferring genes (e.g., IL-2, IFN-γ, and TGF-β) into skin and mucosa by taking advantage of antigen presenting cells (APC) present at high concentration (WO95/05853), rotaviral vaccine through oral administration of gene into mucosa (J. Virol. 72(7): 5757-5761 (1998)), mucosal administration for treatment of autoimmune disease (WO97/46253), mucosal administration for prevention of infectious disease (W096/21356), gene transfer into female genital organ mucosa for prevention of sexually transmitted disease or cervical cancer caused by papillomavirus infection (Infect. Immun. 66(1): 322-329 (1998)), and simplified administration and increased safety obtained by mucosal administration (Proc. Am. Assoc. Cancer Res. 36: 86 Meet., 418 (1995)).

Specifically, the methods of this invention, for producing a recombinant retroviral vector, comprise the steps of, for example, (a) providing cells in which a retroviral genome RNA is transcribed, and the gag and pol proteins and a membrane protein that binds to sialic acid are expressed (virus producing cells), and (b) culturing the cells in the presence of a neuraminidase derived from a Gram-positive bacterium, and recovering the produced virus. Retroviral genome RNA may be altered from the wild-type viral genome, so long as it is packaged into viral particles, and integrated into the chromosomes of target cells. For example, it may be RNA having LTR on both ends and comprising the packaging signal sequence inside. Genome RNA may be inserted with a desired gene. Such an inserted foreign gene may be expressed by the promoter activity of LTR, or expressed from another promoter by inserting the promoter inside the genome.

The gag and pol proteins expressed in virus producing cells, and the genome RNA may be derived from other viruses, so long as these are assembled to form a virus. For example, packaging cells expressing viral proteins derived from HIV may be used to package the SIV genome RNA. In such applications, a membrane protein that binds to sialic acid may be expressed in the packaging cells either transiently or constitutively. By culturing the above virus producing cells in the presence of a neuraminidase from a Gram-positive bacterium, retrovirus having a membrane protein that binds to sialic acid in the envelope is released into the culture medium. The culture or culture supernatant may be recovered to obtain the produced virus.

For example, a retroviral vector may be produced in the present invention as follows. The following specific method of producing a viral vector is shown as an example, and one skilled in the art would be able to modify it appropriately.

For expressing a viral envelope protein for pseudotyping of recombinant virus using a plasmid vector, for example, the gene encoding the protein is inserted into the pCAGGS vector (Niwa H. *et al*. Gene 108: 193-200 (1991)) and the like to construct an expression vector. The genes encoding the HA (or HN), F, and M proteins of a negative strand RNA virus (*e*.*g*., Influenza virus or Sendai virus), for example, are inserted into the expression vector. For example, for construction of an expression plasmid for HA protein from Influenza virus (H1N1), the ORF of HA protein is amplified by PCR using the pDREF HisD plasmid (Microbiol. Immunol. 44(8): 677-685 (2000)) as a template, and an appropriate primer pair. Amplified fragment is digested with NotI, and inserted into the NotI site of a vector in which pCAGGS is attached with an XhoI-NotI site. Following this, the pCAGGS-HA plasmid for expression of HA protein is obtained (WO01/92508). Alternatively, a plasmid for expression of a Sendai virus envelope protein or the like may be prepared as follows: the genes encoding F, HN, and M proteins are cut out from pSeV18⁺b(+), the entire genome DNA of Sendai virus Z strain (Hasan M.K. *et al*. J. General Virol. 78: 2813-2820 (1997)), for example, and inserted into the XhoI site of pCAGGS to construct expression vectors for F, HN, and M proteins from Sendai virus (referred to as pCAGGS F, pCAGGS HN, and pCAGGS M, respectively) (WO01/92508).

In producing a retrovirus, the human embryonic kidney derived cell line 293T cells (Proc. Natl. Acad. Sci. USA 90: 8392-8396 (1993)) may be used as the virus producing cells, for example. 293T cells are cultured in Dulbecco's Modified Eagle medium (DMEM, Gibco BRL) containing 10% inactivated fetal bovine serum, for example. Transfection of DNA vector can be performed using LIPOFECTAMINE PLUS (Gibco BRL) or the like according to the attached manufacturer's instructions. For example, 293T cells are plated into 6-well plastic plate at a density of 1x 10⁶ cells/well, and cultured for 48 hours in a CO₂ gas incubator (37°C, 10% CO₂ gas). Thirty minutes before transfection, the culture medium is replaced with 800 µl of DMEM (Gibco BRL) containing 1% bovine serum albumin (Gibco BRL) per well, and then culturing is continued.

The gene transfer vector is not limited to any particular virus; for example, Murine Stem Cell Virus (MSCV; Clontech) (Hawley R.G *et al*. Proc. Natl. Acad. Sci. USA 93: 10297-10302 (1996); Hawley R. G. *et al*. Gene Therapy 1: 136-138 (1994)) may be used. A desired gene to be expressed is inserted into the vector. In terms of the amount of DNA for transfection, 700 ng of the gene transfer vector and 300 ng of the packaging vector (pCL-Eco, pCL-Ampho (MuLV 4070A), both available from IMGENEX) may be used per well (Naviaux, R. K. *et al*., J. Virol. 70: 5701-5705 (1996)), and in addition to these, 200 ng of the above expression vectors for envelope proteins of a negative strand RNA virus may be used alone or in any combination. DNA is dissolved in 100 µl of OptiMEM, to which 6 µl of PLUS reagent (Gibco BRL) is then added, and after mixing, the solution is incubated still for 15 minutes at room temperature. To the mixture of DNA and PLUS reagent (Gibco BRL), 4 µl of LIPOFECTAMINE diluted in 100 µl of OptiMEM is added, and after mixing, the mixture is incubated for additional 15 minutes at room temperature. A solution containing a complex of DNA and LIPOFECTAMINE prepared as described above is added dropwise to the culture of 293T cells in a 6-well plate, and then mixed gently, and the culture is incubated for 3 hours in a CO₂ gas incubator (37°C, 10% CO₂ gas). Then, 1 ml of DMEM (Gibco BRL) containing 1% bovine serum albumin (Gibco BRL) and 15 µg/ml of trypsin (Gibco BRL) per well is added to the culture, and incubation is continued for additional 24 hours in a CO₂ gas incubator (37°C, 10% CO₂ gas). Subsequently, the culture medium is replaced with 2 ml of DMEM containing 1% bovine serum albumin, 5 µg/ml of trypsin (Gibco BRL), and 0.01 U of neuraminidase from a Gram-positive bacterium for each well, and the culture is continued for additional 24 hr. Then, the culture supernatant is collected, and filtrated through a filter of a pore size 0.45 µm (*e.g*., DISMIC-25CS filter (ADVANTEC)) to obtain vector solution. Neuraminidase from a Gram-positive bacterium may be NA derived from actinomycetes, for example, and NA from *M. viridifaciens*, specifically. The amount ofNA may be appropriately adjusted. Pseudotyped retrovirus with an envelope protein of a negative strand RNA virus having the HA activity has a strong infectivity in mucous cells, such as lung bronchial mucoepithelial cells. Thus, such a vector produced as above is useful for gene transfer into mucous cells. Furthermore, such a viral vector, capable of transferring genes into both human bone marrow CD34 positive cells and CD34 negative cells, is useful for transferring genes into hematopoietic cells.

In addition, a pseudotyped retroviral vector based on Moloney murine sarcoma virus may be suitably produced in the present invention. Moreover, a vector further comprising VSV-G protein as an envelope protein may be prepared. For example, using the above pMSCV EGFP, or the pLZRNL vector expressing LacZ under the control of Moloney murine sarcoma virus-derived LTR (Yee J.-K. *et al*. Methods In Cell Biology 43: 99-112 (1994); Xu L. *et al*. Virology 171: 331-341 (1989)) as the gene transfer vector, pseudotyped retroviral vectors with a variety of envelope proteins may be prepared as described below.

293T cells (human embryonic kidney derived cell line) are cultured in high glucose DMEM (Gibco BRL) containing 10% inactivated bovine calf serum (BIO WHITTAKER) at 37°C, 10% CO₂ gas. The cells are plated into 6-well plastic plates at a density of 5x 10⁵ cells/well, and cultured for 48 hours at 37°C, 10% CO₂ gas. Then, the culture medium is replaced with 800 µl of DMEM containing 1% bovine serum albumin per well, and the cells are subjected to transfection. For each well, 700 ng of the gene transfer vector (pMSCV EGFP, or pLZRNL) is mixed with 100 ng of VSV-G expression plasmid pVSV-G (derived from the Indiana serotype strain; Clontech), 200 ng each of the expression plasmids (constructed with pCAGGS) for HA (or HN), F, and M proteins of a negative strand RNA virus, and 300 ng of the expression plasmids for murine retrovirus capsid protein, pCL-Eco, and pCL-Ampho (Imgenex) (Naviaux R.K. *et al*. J. Virol. 70: 5701-5705 (1996)) in any combination, and dissolved in 100 µl of OptiMEM. After addition of 6 µl of PLUS Reagent (Gibco BRL), the mixture is stirred, and incubated still for 15 minutes at room temperature. To the mixture, 4 µl of LIPOFECTAMINE Reagent (Gibco BRL) diluted in 100 µl of OptiMEM is added and mixed well, and further incubated at room temperature for 15 min. Then, the mixture is added dropwise to the above culture of 293T cells, mixed gently, and the culture is incubated for 3 hours at 37°C, 10% CO₂ gas. Then, 1 ml of DMEM containing 1% bovine serum albumin and 15 µg/ml of trypsin (Gibco BRL) is added to the culture in each well, and incubation is continued for additional 24 hours at 37°C, 10% CO₂ gas. Subsequently, the culture medium of each well is replaced with 2 ml of DMEM containing 1% bovine serum albumin, 5 µg/ml of trypsin (Gibco BRL), and 0.01 U of neuraminidase from a Gram-positive bacterium (actinomycete, for example), and the culture is continued for additional 24 hr. Then, the culture supernatant is collected, and filtrated through a filter of a pore size 0.45 µm to obtain a vector solution.

Furthermore, this invention may be particularly useful for the production of a lentivirus vector. An example of producing a pseudotyped lentiviral vector with VSV-G protein is shown below.

For construction of vectors, the SIVagm TYO-1 strain, a nonpathogenic clone of African green monkey immunodeficiency virus, may be used, for example. Plasmids carrying the SIVagm TYO-1 as an insert may be constructed from pSA212 (J. Virol. 64: 307-312 (1990)), for example. Construction of the gene transfer vector and packaging vector may be performed using the publicly known literatures as a reference (WO01/92508). Specifically, an expression plasmid for providing essential proteins for the formation of virion, in which each of the gag, pol, tat, rev, vif, vpr/x sequence is located downstream of the promoter (packaging vector), is constructed. To avoid production of the wild-type virus, it is preferred to remove the most part of the packaging signal Ψ and env. The SD sequence and RRE sequence may be inserted upstream of gag, and downstream of the first exon of tat/rev, respectively, to express all the genes in the packaging vector. Furthermore, the entire nef sequence, which is considered non-essential for vector packaging, may be deleted.

The gene transfer vector, providing RNA that is packaged in the vector, is constructed to carry the LTR sequence of both ends of the genome, SD sequence, Ψ, and RRE as inserts. Furthermore, the 5'-LTR promoter region of the gene transfer vector may be substituted with a foreign promoter. Furthermore, the sequence of 3'-LTR may be partially deleted to prepare a Self Inactivating Vector (SIN vector) that prevents transcription of mRNA encoding the entire vector in target cells. For such SIN vector, the CMV promoter may be inserted as the promoter, for example, and a desired foreign gene is inserted downstream of it.

VSV-G expression vectors can be used as vectors that provide capsid proteins to form pseudotyped vector particles. For example, the vector used to provide VSV-G may be the field-proven pVSV-G in pseudotyping retroviral and HIV vectors (Bums, J. C. (1993) Proc. Natl. Acad. Sci. USA 90: 8033-8037).

More specific description is provided below.

### <Construction of packaging vectors>

A DNA fragment corresponding to the region (5337 to 5770) containing vif and the first exon of tat/rev is prepared by PCR using an appropriate pair of primers and pSA212 as a template. A site for the restriction enzyme EcoRI is added to one of the PCR primers, and thus the prepared DNA fragment contains an EcoRI site at its 3' end. The PCR fragment is digested with BglII and EcoRI, and purified by using agarose gel electrophoresis and Wizard PCR Preps DNA Purification System (Promega). The DNA fragment prepared as described above and another DNA fragment encoding the gag/pol region (from XhoI site (356) to BglII site (5338)) are ligated into pBluescript KS+ (Stratagene) between the XhoI and EcoRI sites. Then, a Rev responsive element (RRE) and a DNA fragment corresponding to the region containing the second exon (6964 to 7993) of tat/rev are amplified by PCR. ANotI site is added to the 3' end by PCR using, for example, pSA212 as a template. The resulting PCR fragment is double-digested with EcoRI and NotI, followed by purification. The fragment is inserted into pBluescript KS+ containing gag-tat/rev between the EcoRI and NotI sites.

A DNA fragment comprising a sequence for splicing donor (SD) site, which is synthesized to have an XhoI site and SalI site at 5' and 3' ends, respectively, is inserted at the XhoI site of the above-described pBluescript KS+ containing gag-RRE-tat/rev. The resulting plasmid is digested with XhoI and NotI. The fragment containing SD-gag-RRE-tat/rev is purified. A plasmid is prepared by inserting an XhoI/NotI linker into pCAGGS (Gene, vol.108, pp.193-200, 1991) at the EcoRI site, and then the above-mentioned SD- gag-RRE-tat/rev fragment is inserted at the XhoI-NotI site. The plasmid obtained by the method as described above is used as the packaging vector pCAGGS/SIVagm gag-tat/rev (WO01/92508).

### <Construction of gene transfer vectors>

Using pSA212 as a template, PCR is carried out to amplify the SIVagm TYO1-derived 5' LTR region (8547 to 9053 + 1 to 982; containing KpnI site and EcoRI site at the 5' and 3' ends, respectively); the 3' LTR region (8521 to 9170; containing NotI and BamHI sites at the 5' end and SacI site at the 3' end); and the RRE sequence (7380 to 7993; containing EcoRI and SacII sites at the 5' and 3' ends, respectively), using appropriate pairs of primers. The CMV promoter region (1 to 600; containing SacII and NotI sites at the 5' and 3' ends, respectively) derived from pEGFPN2 (Clontech) is amplified using an appropriate pair of primers. The DNA fragments are digested at their ends. After purification, the 5' LTR, RRE, CMV promoter, and 3' LTR are inserted in this order into pBluescript KS+ at the KpnI-SacI site by ligation. For example, a NotI fragment containing the β-galactosidase gene derived from pCM 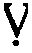 β (Clontech) is inserted as a reporter gene into the NotI site. The resulting plasmid is digested with KpnI and SacI to obtain a DNA fragment containing the region from the 5' LTR to the 3' LTR. The fragment is inserted into a control vector pGL3 (Promega) at the KpnI-SacI site. The resulting plasmid is used as the gene transfer vector pGL3C/5' LTR.U3G2 /RREc/s/CMV F β-gal/WT 3' LTR.

Further, the CMV promoter region derived from pEGFPC2 (Clontech) and the region encoding EGFP (1 to 1330; containing a SacII site at the 5' end and NotI, BamHI sites, and translational stop codon at the 3' end) are amplified by PCR using an appropriate pair of primers and using pEGFPC2 as a template. The four types of PCR fragments are digested with restriction enzymes KpnI and EcoRI, EcoRI and SacII, BamHI and SacI, and SacII and BamHI, respectively. After being purified, the fragments of 5' LTR, RRE, CMV promoter EGFP, and 3' LTR are inserted in this order into pBluescript KS+ between KpnI and SacI by ligation (pBS/5'LTR.U3G2/RREc/s/CMVFEGFP/WT3'LTR). The plasmid pBS/5'LTR.U3G2/RREc/s/CMVFEGFP/WT3'LTR is digested with KpnI and SacI to prepare a DNA fragment containing the region from the 5'LTR to the 3'LTR. The fragment is inserted into pGL3 (Promega) as a control vector at the KpnI-SacI site to construct a vector (pGL3C/5'LTR.U3G2/RREc/s/CMVFEGFP/WT3'LTR).

### <Modification of 5' LTR>

A fragment containing gag region (9039 to 9170 + 1 to 982) downstream of the TATA box of 5' LTR is amplified by PCR using an appropriate pair of primers and using pSA212 as a template. The CMV L promoter of cytomegalovirus (derived from pCI (Promega); nucleotides 1 to 721) is amplified by PCR. A fragment containing a region downstream of the TATA box of 5' LTR is combined with the fragment containing the promoter. A DNA fragment containing a chimeric promoter of the promoter and the 5' LTR is prepared by PCR using the mixture as a template and a primer placed on the 5' side of the promoter and another primer located on the 3' side of 5' LTR. The resulting DNA fragment is inserted into the gene transfer vector (pGL3C/5'LTR.U3G2/RREc/s/CMV 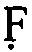 β-gal/WT3'LTR) at the KpnI-EcoRI site to prepare pGL3C/CMVL.U3G2/RREc/s/CMV 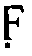 β-gal/WT3'LTR. Similarly, the DNA fragment obtained by the PCR experiment described above is also inserted into the vector pGL3C/5'LTR.U3G2/RREc/s/CMVFEGFP/WT3'LTR at the KpnI-EcoRI site to prepare pGL3C/CMVL.U3G2/RREc/s/CMVFEGFP/WT3'LTR.

### <Preparation of 3' LTR-modified SIN vector (self inactivating vector)>

A DNA fragment containing the 27 bp at the 5' end, 15 bp at the 3' end, and R region from the U3 region of 3' LTR is amplified by PCR using an appropriate pair of primers and using pSA212 as a template. This fragment is inserted into the SalI-SacI site of the gene transfer vector pGL3C/CMVL.U3G2/RREc/s/CMV 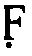 β-gal/WT3'LTR, which is prepared to contain the chimeric promoter in the above Section, to prepare pGL3C/CMVL.U3G2/RREc/s/CMV 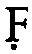 β-gal/3'LTRΔU3. Similarly, this fragment is also inserted into pGL3C/CMVL.U3G2/RREc/s/CMVFEGFP/WT3'LTR at the SalI-SacI site to prepare pGL3C/CMVL.U3G2/RREc/s/CMVFEGFP/3LTRΔU3 (WO01/92508).

The constructed plasmid is transformed into DH5α (Toyobo) by the conventional method, and incubated in agar plates. PCR is carried out using the emerging colonies as templates to confirm the correct structure. Clones confirmed are cultured in 100 ml of LB culture medium. The plasmids are purified with a QIAGEN Plasmid Maxi Kit (QIAGEN).

### <Recovery of viral vector>

293T cells are plated into 6-well plastic plates at a density of 5x 10⁵ cells/well, and cultured for 48 hours at 37°C, 10% CO₂ gas. Then, the culture medium is replaced with 800 µl of OptiMEM (containing 1% BSA if HA is used) per well for the use in transfection. For each well, 600 ng of the above gene transfer vector and 300 ng of the above packaging vector are mixed with expression plasmids for envelope proteins, such as 100 to 300 ng of expression plasmids for HA (or HN) and F proteins of a negative strand RNA virus (constructed in pCAGGS), and 100 ng of the expression plasmid for the VSV-G protein (pVSV-G; Clontech), in any combination, and dissolved in 100 µl of OptiMEM. After addition of 6 µl of PLUS Reagent (Gibco BRL), the mixture is stirred, and incubated still for 15 minutes at room temperature. To the mixture, 100 µl of OptiMEM mixed with 4 µl of LIPOFECTAMINE reagent (Gibco BRL) is added, mixed well, and incubated for additional 15 minutes at room temperature. Then, the mixture is added dropwise to the above culture of 293T cells, mixed gently, and incubated for 3 hours at 37°C, 10% CO₂. Then, 1 ml of DMEM containing 20% inactivated bovine serum (or containing 1% BSA and 5 µg/ml of trypsin instead if HA is used) is added to the culture of each well, and the culture is continued for additional 12 hours at 37°C, 10% CO₂ gas. Subsequently, the culture medium of each well is replaced with 2 ml of DMEM containing 10% inactivated bovine serum (or containing 1% bovine serum albumin and 5 µg/ml of trypsin, instead, with the presence of NA derived from *M viridifaciens* if HA is used), and the culture is continued for 24 hr. Then, the culture supernatant is recovered, and filtrated through a filter with a pore size 0.45 µm.

### <SIVagm vector-mediated gene transfer>

293T cells are plated in a 6-well plastic culture plate at a cell density of 5x 10⁵ cells/well, and incubated under 10% CO₂ at 37°Cfor 48 hours. The culture medium is removed, and 1 ml of the solution containing the vector solution to which polybrene (Sigma) is added at a final concentration of 8 µg/ml is overlaid onto the cells. The cells are incubated under 10% CO₂ at 37°C for 3 hours to achieve vector transfect. After three hours, 1 ml of the culture medium is added to the cells. On the following day, the culture medium is changed. Next day, when the vector used is the β-Gal expression vector, staining is carried out using X-gal as the substrate with a β-Gal Staining Kit (Invitrogen), and the expression of β-galactosidase in the target cells is observed under a light microscope. However, when the vector used is the EGFP expression vector, the expression is analyzed under a fluorescence microscope.

### <Vector titration>

Vector titration is carried out by calculating the number of cells into which a gene is introduced using 1 ml of the vector solution. 293T cells are plated in a 6-well plastic culture plate at a cell density of 1x 10⁶ cells/plate, and incubated for 48 to 72 hours. By the same procedure as described above, infection to the cells is carried out with serially diluted vector solutions. X-gal staining is performed 48 hours after infection. For example, the mean value for the number of cells containing the transferred gene in a visual field is determined from three different visual fields under a light microscope with 200-fold magnification, and multiplied by the coefficient 854.865 that has been determined based on the area of the visual field and the area of the plate to determine the titer. The unit of titer of viral vector thus calculated is defined as Transducing Unit (T.U.)/ml.

The SIVagm vector thus prepared mediates gene transfer into culture cells and other in *vivo* and *ex vivo* cells highly efficiently. The probability of reconstitution of wild-type viral particles is assumed to be very low in vector packaging after co-transfection of a number of independent plasmids, such as the above-described gene transfer vector, packaging vector, and envelope expression vector. Furthermore, SIVagm TYO-1 itself, used as a base for the vector, has been confirmed to exhibit no pathogenicity in terms of both natural and experiment infection (Ohta, Y. *et al*., Int. J. Cancer: 41, 115-22, 1988; Miura, T. *et al*., J. Med. Primatol.: 18(3-4), 255-9. 1989; Honjo, S. *et al*., J. Med. Primatol. 19(1), 9-20, 1990). In addition, the vector can be highly safe because generally lentiviruses are highly species-specific and have only weak pathogenicity to animal species other than their original target species (Novembre, F. J. *et al*., J. Virol.: 71(5), 4086-91. 1997).

In this vector producing system, the packaging signal sequence has been removed from the packaging vector construct, and thus the RNA encoding viral proteins is not packaged into particles. The binding of rev protein to RRE induces transfer of RNA into the cytoplasm and suppression of splicing, resulting in the expression of viral proteins and packaging of full-length RNA into viral particles. Therefore, the inserted RRE in both packaging vector and gene transfer vector can regulate mRNA splicing of the packaging vector, which thus can allow expression of all genes. Then, mRNA of the gene transfer vector can be transferred into the cytoplasm, and packaged into the vector particles. There are some cases where *vif* and *vpr*/*x* have been excluded from the HIV-1 vectors (Dull, T. *et al*., J. Virol., 72(11), 8463-71. 1998), and this suggests the possibility that proteins are not essential for packaging and functioning of vector particles. *vpr* is believed to be a factor responsible for the infectivity to nondividing cells (Heinzinger, N. K. *et al*., Proc. Natl. Acad. Sci. U S A, 91(15), 7311-5, 1994); a report describes that the type of cell, to which HIV-1 vector can transfer genes, varies depending on the presence of *vpr* (Kim, V. N. *et al*., J. Virol., 72(1), 811-6. 1998). It has also been reported that *nef*, which was completely excluded form the packaging vector as described above, can be a causative protein of the SIV-mediated immunodeficiency based on the evidence obtained by experiments with infection to monkey (von Gegerfelt, A. S. *et al*., J. Virol. 73, 6159-65, 1999; Kestler, H. W. 3d., Naidu, Y. N., Kodama, T., King, N. W., Daniel, M. D., Li, Y., Desrosiers, R. C. Use of infectious molecular clones of simian immunodeficiency virus for pathogenesis studies., J. Med. Primatol. 18(3-4): 305-9, 1989). The corresponding sequence has been removed completely from the SIVagm vector constructed as described above; therefore, even if reconstituted viral particles containing viral genes derived from the packaging vector were formed, the risk of pathogenicity for such particles would be further decreased.

The lentivirus-based vector can transfer genes into cell-cycle arrested culture cells and neurons because the original lentivirus is infectious to nondividing cells (Naldini, L. *et al*., Science: 272 263-267, 1996; Sutton, R. E. *et al*., J. Virol., 73(5), 3649-60, 1999). When such a vector is pseudotyped by VSV-G, unlike the original SIV, the infectivity of the vector is not limited to the infection to CD4- and chemokine receptor-positive cells. The receptor of VSV-G is known to be phosphatidylserine, which is one of the phospholipids, whose molecules are present on the surface of various types of cells (Schlegel, R. *et al*., Cell, 32(2), 639-46, 1983). Thus, the SIVagm vector which has been pseudotyped by VSV-G has a considerably wider range of infectivity. It is predicted that when viruses that are pseudotyped by membrane proteins having sialic acid binding activity are prepared based on this vector, they are capable of transferring genes to almost all types of animal cells with a high degree of efficiency.

The amount of plasmid vectors used for transfection into cells in preparation of viral vector may be adjusted appropriately. For example, the following method may be used, although it is not limited thereto.

### 1. Cell culture

293T cells (human embryonic kidney derived cell line) are cultured in high glucose DMEM (Gibco BRL) containing 10% inactivated bovine calf serum (BIO WHITTAKER) at 37°C, 10% CO₂.

### 2. Construction of vector

293 T cells are plated into 6-well plastic plates at a density of 5x 10⁵ cells/well, and cultured for 48 hours at 37°C, 10% CO₂. Then, the culture medium in each well is replaced with 800 µl of DMEM containing 1% bovine serum albumin for use in transfection. For each well, 1200 ng of the gene transfer vector (pGL3C/CMVL.U3G2/RREc/s/CMVF EGFP/3LTRΔU3, or pGL3C/CMVL.U3G2/RREc/s/CMVF β-gal/3LTRΔU3) and 360 ng of the packaging vector (pCAGGS/SIVagm gag-tat/rev) are mixed with any combination of expression plasmids for envelope proteins: 120 ng of VSV-G expression plasmid pVSV-G (Clontech), and 240 ng each of expression plasmids for HA (or HN), F, and M proteins of a negative strand RNA virus (in pCAGGS), and dissolved in 100 µl of OptiMEM. After addition of 6 µl of PLUS Reagent (Gibco BRL), the mixture is stirred, and incubated still for 15 minutes at room temperature. Four µl of LIPOFECTAMINE reagent (Gibco BRL) diluted in 100 µl of OptiMEM is added to the mixture, mixed well, and incubated for additional 15 minutes at room temperature. Then, the mixture is added dropwise to the above culture of 293T cells, mixed gently, and incubated for 3 hours at 37°C, 10% CO₂. Then, 1 ml of DMEM containing 1% BSA and 15 µg/ml of trypsin (Gibco BRL) (10 µg/ml of trypsin, instead, if HA is used) is added to the culture of each well, and incubated for 24 hours at 37°C, 10% CO₂. Subsequently, the culture medium of each well is replaced with 2 ml of DMEM containing 1% bovine serum albumin, 7.5 µg/ml of trypsin (Gibco BRL) (5 µg/ml trypsin for HA), and 0.01 U ofNA derived from a Gram-positive bacterium (actinomycete, for example), and the culture is continued for additional 24 hr. Then, the culture supernatant is recovered, and filtrated through a filter of a pore size 0.45 µm to obtain a vector solution.

Large-scale preparation of vector and its concentration are performed as follows. For example, 293T cells are plated into 15 cm-diameter plastic petri dishes at 5x 10⁶ cells/dish, and cultured for 48 hours at 37°C, 10% CO₂. The culture medium of each dish is replaced with 10 ml of DMEM containing 1% bovine serum albumin, and the culture is subjected to transfection. For each dish, 8 µg of the gene transfer vector and 2.4 µg of the packaging vector are mixed with any combination of 0.8 µg of VSV-G expression plasmid pVSV-G (Clontech), and 1.6 µg each of expression plasmids for HA (or HN) and F proteins of a negative strand RNA virus (in pCAGGS), and dissolved in 1.5 ml of OptiMEM. 40 µl of PLUS Reagent (Gibco BRL) is added to the plasmid solution, and mixed, and the mixture is incubated still for 15 minutes at room temperature. 60 µl of LIPOFECTAMINE reagent (Gibco BRL) diluted in 1.5 ml of OptiMEM is added to the mixture, mixed well, and incubated for additional 15 minutes at room temperature. Then, the mixture is added dropwise to the above culture of 293T cells, mixed gently, and incubated for 3 hours at 37°C, 10% CO₂. Then, 10 ml of DMEM containing 1% BSA and 15 µg/ml of trypsin (Gibco BRL) is added to the culture in each dish, and incubated for 24 hours at 37°C, 10% CO₂. Subsequently, the culture medium of each dish is replaced with 20 ml of DMEM containing 1% bovine serum albumin, 7.5 µg/ml of trypsin (Gibco BRL) (5 µg/ml of trypsin, if HA is used), and approximately 0.05 to 0.5 U ofNA derived from a Gram-positive bacterium (for example, actinomycete). After incubation for additional 24 hours at 37°C, 10% CO₂, the culture supernatant is recovered, filtrated through a filter of a pore size 0.45 µm, and centrifuged at 42,490 x g, 4°C for 90 minutes (16,000 x g if F/HN is used) (TOMY SRX-201, TA21BH). The pellet is dissolved in PBS (containing 5% FCS, 2 µg/ml polybrene), and stored at -80°C until use.

In another example, a pseudotype lentiviral vector with Influenza HA protein can be concentrated. Concentration may be performed as follows. 293T cells are plated into 15 cm-diameter plastic petri dishes at 5x 10⁶ cells/dish, and cultured for 48 hours at 37°C, 10% CO₂. The culture medium of each dish is replaced with 10 ml of DMEM containing 1% bovine serum albumin, and the culture is subjected to transfection. For each dish, 8 µg of the gene transfer vector (pGL3C/CMVL.U3G2/RREc/s/CMVF LacZ/3LTRΔU3) is mixed with 2.4 µg of the packaging vector (pCAGGS/SIVagm gag-tat/rev) and 1.6 µg of Influenza HA protein expression plasmid pCAGGS-HA, and dissolved in 1.5 ml of OptiMEM (Gibco BRL). 40 µl of PLUS Reagent (Gibco BRL) is added to the plasmid solution, mixed, and incubated still for 15 minutes at room temperature. 60 µl of LIPOFECTAMINE reagent (Gibco BRL) diluted in 1.5 ml of OptiMEM is added to the mixture, mixed well, and incubated for additional 15 minutes at room temperature. Then, the mixture is added dropwise to the above culture of 293T cells, mixed gently, and incubated for 3 hours at 37°C, 10% CO₂. Then, 10 ml of DMEM containing 1% BSA and 10 µg/ml of trypsin (Gibco BRL) is added to the culture in each dish, and incubated for 16 to 24 hours at 37°C, 10% CO₂. Subsequently, the culture medium of each dish is replaced with 20 ml of DMEM containing 1% bovine serum albumin, 5 µg/ml of trypsin (Gibco BRL), and approximately 0.05 to 0.5 U ofNA derived from a Gram-positive bacterium (actinomycete, for example). After incubation for additional 24 hr, the culture supernatant is recovered, filtrated through a filter of a pore size 0.45 µm, and centrifuged at 16,000 x g, 4°C for 1 hour (Beckman J-25I, JA-18). The pellet is resuspended in PBS (containing 5% FCS, 2 µg/ml polybrene), and stored at -80°C. Use of Influenza virus HA enables to perform gene transfer without co-expression of other envelope proteins such as VSV-G. HA pseudotype vectors can be highly concentrated by centrifugation.

In addition, a pseudotyped lentiviral vector may be prepared using two or more kinds of sialic acid binding proteins. Below described is an example of producing pseudotype lentiviral vector with envelope proteins of Influenza virus and Sendai virus.

### <Cell culture>

293T cells (human embryonic kidney derived cell line) are cultured in high glucose DMEM (Gibco BRL) containing 10% inactivated bovine calf serum (BIO WHITTAKER) at 37°C, 10% CO₂.

### <Construction of vector>

293T cells are plated into 6-well plastic plates at 5x 10⁵ cells/well, and cultured for 48 hours at 37°C, 10% CO₂. The culture medium in each well is replaced with 800 µl of DMEM containing 1% bovine serum albumin, and the culture is subjected to use in transfection. For each well, 1200 ng of the gene transfer vector (pGL3C/CMVL.U3G2/RREc/s/CMVF EGFP/3LTRΔU3), 360 ng of the packaging vector (pCAGGS/SIVagm gag-tat/rev), and 240 ng each of expression plasmids for Sendai virus HN protein (pCAGGS-HN) and HA protein are mixed, and dissolved in 100 µl of OptiMEM (Gibco BRL). Six µl of PLUS Reagent (Gibco BRL) is added to the plasmid solution, mixed, and incubated still for 15 minutes at room temperature. Four µl of LIPOFECTAMINE reagent (Gibco BRL) diluted in 100 µl of OptiMEM is added to the mixture, mixed well, and incubated for additional 15 minutes at room temperature. Then, the mixture is added dropwise to the above culture of 293T cells, mixed gently, and incubated for 3 hours at 37°C, 10% CO₂. Then, 1 ml of DMEM containing 1% BSA and 10 µg/ml of trypsin (Gibco BRL) is added to the culture of each well, and incubated for 16 to 24 hours at 37°C, 10% CO₂. Subsequently, the culture medium of each well is replaced with 2 ml of DMEM containing 1% bovine serum albumin and 5 µg/ml of trypsin (Gibco BRL), and the culture is continued for 24 hr. Furthermore, the culture medium of each well is replaced with 2 ml of DMEM containing 1% bovine serum albumin, 5 µg/ml of trypsin (Gibco BRL), and 0.01 U of NA derived from a Gram-positive bacterium (actinomycete, for example), and the culture is continued for additional 24 hr. Then, the culture supernatant is recovered, and filtrated through a filter of a pore size 0.45 µm to obtain a vector solution.

Vectors that are produced by the method of the present invention can be purified by a commonly known method of virus purification. Purification may be performed by publicly known methods of purification and separation, such as the above centrifugation, filtration, adsorption, and column purification, or combination of those. Thus, a viral vector comprising a membrane protein that binds to sialic acid that is substantially pure can be obtained. Herein, a substantially pure viral vector means that the viral vector is substantially free of other viral vectors capable of transferring nucleic acid into the cell. A substantially pure viral vector is preferably free of any debris of virus producing cells or other contaminants.

A viral vector produced by the method of the present invention may be provided as a composition, by appropriately combining it with a pharmaceutically acceptable carrier or vehicle. Herein, the phrase "pharmaceutically acceptable carrier or vehicle" means a substance that can be administered together with a vector without inhibiting the vector-mediated gene transfer significantly. Specifically, for example, sterilized water, saline, culture medium, serum, or phosphate-buffered saline (PBS) may be used to mix with a vector for pharmaceutical manufacturing. Furthermore, other substances, such as stabilizers or antibiotics, may be optionally included as well. The composition of the present invention may be present in a form of aqueous solution, capsule, suspension, syrup, or so. Such a composition, comprising a viral vector produced by the method of this invention, is useful as a regent or medicine. For example, the composition may be useful as a reagent for gene transfer into a variety of cells *in vivo* or *in vitro,* or a medicine for gene therapy *ex vivo* or *in vivo.* In general, the composition is administered to a patient by a method commonly known to one skilled in the art: for example, arterial injection, intravenous injection, intraperitoneal injection, subcutaneous injection, enteral administration, oral administration, nasal administration, or *ex vivo* administration. In particular, it is suitable for administration into the nasal or bronchial mucosa, and *ex vivo* administration into blood cells and hematopoietic cells. The amount of administration for the vector may vary depending on disease, patient's body weight, age, sex, symptoms, the objective of administration, formulation of administered composition, administration method, and gene to be transferred, but one skilled in the art should be able to determine appropriately.

A viral vector produced by the method of the present invention may be applied to gene therapy for a variety of genetic diseases. The target disease is not limited to any particular one. Examples of potential target diseases and their single responsible genes include Gaucher's disease, β-cerebrosidase (chromosome 20); hemophilia, coagulation factor VIII (chromosome X) and coagulation factor IX (chromosome X); adenosine deaminase deficiency, adenosine deaminase; phenylketonuria, phenylalanine hydroxylase (chromosome 12); Duchenne type muscular dystrophy, dystrophin (chromosome X); familial hypercholesterolemia, LDL receptor (chromosome 19); and cystic fibrosis, CFTR gene. Such genes may be integrated into chromosome using the viral vector of this invention. In addition, potential target diseases in which multiple genes are implicated include neurodegenerative disorders such as Alzheimer's disease and Parkinson's disease, ischemic encephalopathy, dementia, and intractable infectious diseases such as acquired immunodeficiency syndrome (AIDS). It is possible to perform a gene therapy by taking hematopoietic stem cells out of the body of an AIDS patient, introducing a SIV-based vector produced by the method of this invention *in vitro*, boosting the transcription from the genome derived from SIV before HIV infection occurs, putting back the cells into the patient body, and thus obliterating transcription factors of HIV. Furthermore, in applications for treating a chronic disease, the vector may be used for suppressing expression of VEGF and FGF-2 genes in ischemic heart disease, or cell proliferation related genes such as growth factors (e.g., PDGF and TGF-β), cyclin-dependent kinase, or the like for gene therapy of arteriosclerosis. In diabetes, BDNF gene may be a candidate. In addition, the method enables applications such as complementation therapy in which a gene encoding a tumor suppressor whose mutation causes cancer, such as p53 gene, is integrated into chromosomes, or a treatment surpassing the limitation of drug therapy for cancer by introducing a multi-drug resistance gene into bone marrow hematopoietic stem cells *in vitro*, and putting back into the patient blood. For gene therapy of autoimmune diseases such as multiple sclerosis, rheumatoid arthritis, SLE, and glomerular nephritis, it is possible to suppress expression of T cell receptor, a variety of adhesion molecules (e.g., ICAM-1, LFA-1, VCAM-1, and LFA-4), cytokines and cytokine receptors *(e.g.,* TNF, IL-8, IL-6, and IL-1), growth factors (*e*.*g*., PDGF and TGF-β), effector molecules (*e.g.,* MMP) by antisense expression. For gene therapy of allergic diseases, it is possible to suppress expression of IL-4, FcεR-I, and such by antisense expression. For gene therapy related to tissue transplantation, it is possible to improve the success rate of heteroplasty by humanizing the histocompatibility antigen of non-human animal donor. Furthermore, it is possible to assist therapeutically a shortage of circulating enzymes, growth factors, or such by introducing a foreign gene into chromosomes of human ES cells, and thereby complementing those genes that are otherwise deficient in embryos.

For example, interleukin-4 (IL-4) promotes differentiation of helper T lymphocytes to Th2 lymphocytes. Th2 lymphocytes, in turn, secrete IL-4, IL-5, IL-9, and IL-13, cytokines mediating inflammation associated with asthma. IL-4 is one of the molecules implicated in respiration disorder that induce mucosal secretion from lung mucous membrane. IL-4 regulates the expression of VCAM-1, cell adhesion molecule that interacts with VLA 4 molecule present in the surface of eosinophiles. Through this interaction, eosinophiles can migrate from blood stream to the inflammation site in the lung tissues. IL-4 induces potentiation of B cells and production of antigen specific IgE required for triggering allergic reactions. Antigen specific IgE induces from mast cells release of inflammatory mediators such as histamine and leukotriene, which cause bronchial contraction. Because of these roles of IL-4, a vector expressing a soluble IL-4 receptor may be useful for therapy targeted at asthma patients.

### Brief Description of the Drawings

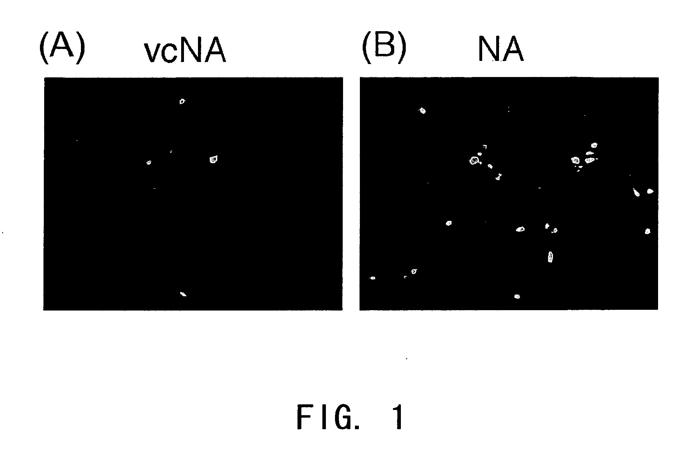

Fig. 1 consists of a pair of photographs showing the result of gene transfer using virus produced using NA derived from *Micromonospora viridifaciens* (NA, right). As a control, virus was produced using NA from *Vibrio cholerae* otherwise similarly, and the same volume of culture supernatant of virus producing cells was used for gene transfection (vcNA, left).

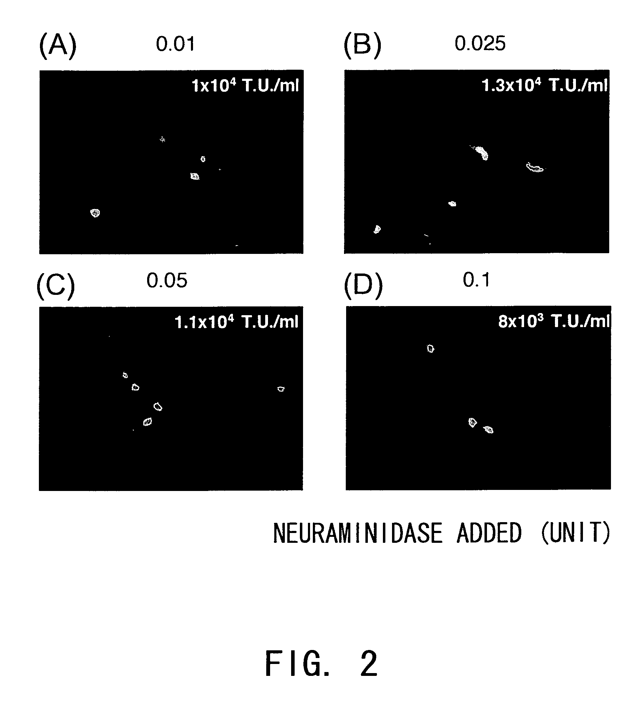

Fig. 2 consists of a set of photographs showing the titer of virus produced using different amounts of NA from *Vibrio cholerae*. Gene transfection was performed using the same volume of culture supernatant of virus producing cells under the same conditions. The number above each panel indicates the amount of NA added (unit).

### Best Mode for Carrying out the Invention

The present invention is explained in detail below with reference to examples, but should not to be construed as being limited thereto. The references cited herein are incorporated into this description as its part.

### [Example 1] Preparation of pseudotyped SIV vector with influenza viral envelope using NA derived from Gram-positive bacterium

### Cell culture

293T cells (human embryonic kidney derived cell line) (Proc. Natl. Acad. Sci. U.S.A. 90: 8392-8396 (1993)) were cultured in DMEM with high glucose (Gibco BRL) supplemented with 10% inactivated bovine calf serum (BIO WHITTAKER) at 37°C, 10% CO₂.

### Vector construction

293T cells were seeded in a 6-well plastic culture plate at 5 x 10⁵ cells/well, and cultured for 48 hours at 37°C, 10% CO₂. The culture medium was replaced with 800 µl of DMEM containing 1% bovine serum albumin in each well, and the cells were subjected to transfection. For each well, 1200 ng of the gene transfer vector pGL3C/CMVL.U3G2/RREc/s/CMVFEGFP/3LTRΔU3, 360 ng of the packaging vector pCAGGS/SIVagm gag-tat/rev, and 240 ng of HA protein expression plasmid pCAGGS-HA were dissolved in 100 µl of Opti MEM (Gibco BRL), then 6 µl of PLUS Reagent (Gibco BRL) was added, mixed, and incubated for 15 minutes at room temperature (WO01/92508). Then, 4 µl of LIPOFECTAMINE (Gibco BRL) diluted in 100 µl of Opti MEM was added to the solution, mixed well, and incubated further for 15 minutes at room temperature. The mixture was added dropwise to the above culture of 293T cells, gently mixed, and the culture was incubated for 3 hours at 37°C, 10% CO₂. One ml of DMEM containing 1% bovine serum albumin and 10 µg/ml of trypsin (Gibco BRL) was added to each well, and the culture was continued for 16 to 24 hours at 37°C, 10% CO₂. Then, the culture medium of each well was replaced with 2 ml of DMEM containing 1% bovine serum albumin, 5 µg/ml of trypsin (Gibco BRL), and 0.01 unit of neuraminidase purified from *M. viridifaciens*. After 24 hours of culture, the culture supernatant was collected, and filtered through 0.45 µm pore filter for subsequent use. As a control, neuraminidase purified from *V. cholerae* (Roche) was used at 0.05 unit to produce vector similarly.

### Gene transfer mediated by SIVagm vector

293T cells, a target cell, were seeded in a 6-well plastic culture plate at 1 x 10⁶ cells/well, and cultured for 48 hours at 37°C, 10% CO₂. The culture medium was removed from the culture, and 1 ml of vector solution supplemented with 8 µg/ml (final) of polybrene (Sigma) was overlaid onto the culture, and incubated for 3 hours at 37°C, 10% CO₂ for vector infection. Then, 1 ml of culture medium supplemented with 20% inactivated bovine calf serum (BIO WHITTAKER) was added to the culture, and the culture was continued for 48 to 72 hours at 37°C, 10% CO₂.

### Titration of vector

The titer of vector was measured by calculating the number of cells that successfully received gene transfer with 1 ml of vector solution. The cells were infected with 1 ml of vector solution as described above, and, after 72 hr, examined on an inverted fluorescence microscope (DMIRB(SLR); Leica) at the magnitude of 200x. The number of gene transfected cells (GFP positive cells) was counted in each field, and the average of three fields was obtained. The average was multiplied by 854.865, a coefficient obtained from the area of a field and plate, to calculate titer. The unit of titer was indicated by Transducing unit (TU)/ml. The result showed that while the titer obtained with NA from *V. cholerae* (VcNA) was 2.8 x 10⁴ (TU/ml), the titer obtained with NA from *M. viridifaciens* (MvNA) was 1.1 x 10⁵, which was significantly higher than that with VcNA (Fig. 1).

### [Example 2] Effect of dosage of added NA from V. cholerae on production of an HA pseudotyped SIV vector

Production of an HA pseudotyped SIV vector was performed as described above with the addition of varying amounts ofNA derived form *V. cholerae*, and the effect was examined. The result showed that viral titers obtained with NA added at 0.01 to 0.1 U (0.005 to 0.05 U/ml) were not significantly different. Thus, the result argues against the possibility that a low titer of vectors produced using NA from *V. cholerae* is due to the low amount of NA used (Fig. 2).

### Industrial Applicability

The present invention provides novel methods for producing a viral vector containing a membrane protein that binds to sialic acid in the envelope, using neuraminidase derived from Gram-positive bacteria. Neuraminidase from Gram-positive bacteria is not only available at low cost, but is also capable of producing a virus with high titer at low dose. Thus, the methods of this invention should achieve excellent cost performance for industrial production of virus in large scale. Produced vectors are suitable for clinical applications, including gene therapy.

## Claims

1. A method for producing a viral vector comprising a membrane protein that binds to sialic acid, comprising the steps of culturing cells producing the viral vector in the presence of a neuraminidase derived from a Gram-positive bacterium, and recovering the produced virus.

2. The method of claim 1, wherein said Gram-positive bacterium is an actinomycete.

3. The method of claim 2, wherein said actinomycete belongs to the Micromonosporaceae family.

4. The method of claim 3, wherein said actinomycete belonging to the Micromonosporaceae family is *Micromonospora viridifaciens*.

5. The method according to any one of claims 1 to 4, wherein said viral vector is a retroviral vector.

6. The method of claim 5, wherein said retroviral vector is a lentiviral vector.

7. The method according to any one of claims 1 to 6, wherein said membrane protein that binds to sialic acid is an envelope protein of a single stranded negative strand RNA virus.

8. The method of claim 7, wherein said single stranded negative strand RNA virus is a virus belonging to the Paramyxoviridae or Orthomyxoviridae family.

9. The method according to any one of claims 1 to 6, wherein said membrane protein that binds to sialic acid is an HA protein of an influenza virus.

10. A virus produced using the method according to any one of claims 1 to 9.


SEQUENCE LISTING

<110> DNAVEC RESEARCH INC.

<120> Methods of producing a viral vector comprising a membrane protein
that binds to sialic acid as a component of the envelope
using neuraminidase derived from Gram-positive bacteria

<130> D3-A0204P

<150> JP 2002-258576
<151> 2002-09-04

<160> 2

<170> PatentIn version 3.1

<210> 1
<211> 1941
<212> DNA
<213> Micromonospora viridifaciens

<220>
<221> CDS
<222> (1)..(1941)
<223>

<400> 1

atg act gcg aat ccg tac ctc cgc cgc ctg ccc cgg cgc cga gcc gtc 48

Met Thr Ala Asn Pro Tyr Leu Arg Arg Leu Pro Arg Arg Arg Ala Val

1               5                   10                  15

agc ttc ctg ctc gca cca gcg ctg gcg gcc gcc acg gtc gcc ggc gcg 96

Ser Phe Leu Leu Ala Pro Ala Leu Ala Ala Ala Thr Val Ala Gly Ala

            20                  25                  30

tcc ccc gca cag gcc atc gcc ggg gca ccc gtc ccg ccc ggc ggc gag 144

Ser Pro Ala Gln Ala Ile Ala Gly Ala Pro Val Pro Pro Gly Gly Glu

        35                  40                  45

ccg ctc tac acg gag cag gac ctc gcc gtg aac ggc agg gag ggc ttt 192

Pro Leu Tyr Thr Glu Gln Asp Leu Ala Val Asn Gly Arg Glu Gly Phe

    50                  55                  60

ccg aac tac cgc atc cca gcg ctg acc gtc acg ccc gac ggg gac ctg 240

Pro Asn Tyr Arg Ile Pro Ala Leu Thr Val Thr Pro Asp Gly Asp Leu

65                  70                  75                  80

ctg gcc tcg tac gac ggc cgc ccg acc ggt atc gac gcg ccc ggc ccc 288

Leu Ala Ser Tyr Asp Gly Arg Pro Thr Gly Ile Asp Ala Pro Gly Pro

                85                  90                  95

aac tcc atc ctc caa cgc cgc agc acc gac ggc ggc cgg acg tgg ggc 336
Asn Ser Ile Leu Gln Arg Arg Ser Thr Asp Gly Gly Arg Thr Trp Gly
            100                 105                 110

gag caa cag gtc gtc agc gcc ggc cag acc acc gcg ccg atc aag ggg 384
Glu Gln Gln Val Val Ser Ala Gly Gln Thr Thr Ala Pro Ile Lys Gly
        115                 120                 125

ttc tcc gac ccc agc tac ctt gtc gac cgg gaa acc ggg acc atc ttc 432
Phe Ser Asp Pro Ser Tyr Leu Val Asp Arg Glu Thr Gly Thr Ile Phe
    130                 135                 140

aac ttc cac gtc tac tcc cag cgg cag ggc ttc gcc ggc agc cgg ccc 480
Asn Phe His Val Tyr Ser Gln Arg Gln Gly Phe Ala Gly Ser Arg Pro
145                 150                 155                 160

ggc acc gac ccg gca gac ccc aac gtg ctc cac gcc aac gtc gcg acc 528
Gly Thr Asp Pro Ala Asp Pro Asn Val Leu His Ala Asn Val Ala Thr
                165                 170                 175

tcg acc gac ggc ggt ctg acc tgg tcg cac cgg acc atc acg gcc gac 576
Ser Thr Asp Gly Gly Leu Thr Trp Ser His Arg Thr Ile Thr Ala Asp
            180                 185                 190

atc acc ccg gat ccg ggc tgg cgc agc cgc ttc gcc gcc tcc ggc gaa 624
Ile Thr Pro Asp Pro Gly Trp Arg Ser Arg Phe Ala Ala Ser Gly Glu

        195                 200                 205

ggc atc cag ctc cgc tat gga ccc cac gcc ggt cga ctc atc cag cag 672
Gly Ile Gln Leu Arg Tyr Gly Pro His Ala Gly Arg Leu Ile Gln Gln
    210                 215                 220

tac acg atc atc aac gct gcc ggc gcc ttc cag gcg gtg agc gtg tac 720
Tyr Thr Ile Ile Asn Ala Ala Gly Ala Phe Gln Ala Val Ser Val Tyr
225                 230                 235                 240

agc gac gac cac gga agg acc tgg cgc gcc ggc gaa gcc gtc ggg gtc 768
Ser Asp Asp His Gly Arg Thr Trp Arg Ala Gly Glu Ala Val Gly Val
                245                 250                 255

ggc atg gac gag aac aag acc gtg gaa ctc tcc gat ggc cgg gtc ctg 816
Gly Met Asp Glu Asn Lys Thr Val Glu Leu Ser Asp Gly Arg Val Leu
            260                 265                 270

ctc aac agc cgc gac tcg gcc cgc agc gga tac cgt aag gtg gcc gtc 864
Leu Asn Ser Arg Asp Ser Ala Arg Ser Gly Tyr Arg Lys Val Ala Val
        275                 280                 285

tcc act gac ggc ggc cac agc tac ggc ccg gtg acc atc gac cgc gac 912
Ser Thr Asp Gly Gly His Ser Tyr Gly Pro Val Thr Ile Asp Arg Asp
    290                 295                 300

ctc ccc gac ccg acg aac aac gca tcg atc atc cgg gcc ttc cct gac 960
Leu Pro Asp Pro Thr Asn Asn Ala Ser Ile Ile Arg Ala Phe Pro Asp
305                 310                 315                 320

gcc ccg gcc ggc tcc gcg cgg gcc aag gtc ctg ctc ttc tcc aac gcc 1008
Ala Pro Ala Gly Ser Ala Arg Ala Lys Val Leu Leu Phe Ser Asn Ala
                325                 330                 335

gcc agc cag acc tcg cgc agt cag ggc acc atc cgg atg tcc tgc gac 1056
Ala Ser Gln Thr Ser Arg Ser Gln Gly Thr Ile Arg Met Ser Cys Asp
            340                 345                 350

gat ggc cag acc tgg ccg gtt tcg aag gtc ttc cag ccc ggc tcg atg 1104
Asp Gly Gln Thr Trp Pro Val Ser Lys Val Phe Gln Pro Gly Ser Met
        355                 360                 365

tcg tac tcc acc ctg acc gca ctg ccc gac ggc acc tac ggg ctg ctg 1152
Ser Tyr Ser Thr Leu Thr Ala Leu Pro Asp Gly Thr Tyr Gly Leu Leu
    370                 375                 380

tac gag ccg ggc acc ggc atc aga tac gcc aac ttc aac ctc gcc tgg 1200
Tyr Glu Pro Gly Thr Gly Ile Arg Tyr Ala Asn Phe Asn Leu Ala Trp
385                 390                 395                 400

ctg ggc ggc atc tgc gcg ccc ttc acg att ccg gat gtg gcg ctc gag 1248
Leu Gly Gly Ile Cys Ala Pro Phe Thr Ile Pro Asp Val Ala Leu Glu

                405                 410                 415

ccg ggc cag cag gtc act gtt ccg gtg gcc gtc acg aac cag tcc ggt 1296
Pro Gly Gln Gln Val Thr Val Pro Val Ala Val Thr Asn Gln Ser Gly
            420                 425                 430

atc gcg gta ccg aag ccg agc ctt cag ctc gac gca tcg ccg gac tgg 1344
Ile Ala Val Pro Lys Pro Ser Leu Gln Leu Asp Ala Ser Pro Asp Trp
        435                 440                 445

cag gtt cag ggt tcc gtc gag ccc ctc atg ccc gga cgg cag gcc aag 1392
Gln Val Gln Gly Ser Val Glu Pro Leu Met Pro Gly Arg Gln Ala Lys
    450                 455                 460

ggc cag gtg acc atc acg gtt ccc gcc ggc acc acc ccc ggt cgc tac 1440
Gly Gln Val Thr Ile Thr Val Pro Ala Gly Thr Thr Pro Gly Arg Tyr
465                 470                 475                 480

cgg gtc ggt gcg acg ctg cgc acc tcc gcg ggt aac gcg tcg acg acc 1488
Arg Val Gly Ala Thr Leu Arg Thr Ser Ala Gly Asn Ala Ser Thr Thr
                485                 490                 495

ttc acg gtc acg gtt gga ctg ctc gac cag gcc cgg atg agc atc gcg 1536
Phe Thr Val Thr Val Gly Leu Leu Asp Gln Ala Arg Met Ser Ile Ala
            500                 505                 510

gac gtc gac agc gag gag acc gcc cgc gaa gac ggg cgg gcg agc aac 1584
Asp Val Asp Ser Glu Glu Thr Ala Arg Glu Asp Gly Arg Ala Ser Asn
        515                 520                 525

gtg atc gac ggc aac ccc tcg acg ttc tgg cac acc gaa tgg tcg cgt 1632
Val Ile Asp Gly Asn Pro Ser Thr Phe Trp His Thr Glu Trp Ser Arg
    530                 535                 540

gcc gat gct cct ggc tac ccg cac cgc atc agc ctc gac ctc ggt ggc 1680
Ala Asp Ala Pro Gly Tyr Pro His Arg Ile Ser Leu Asp Leu Gly Gly
545                 550                 555                 560

acg cac acg atc agc ggc ctc cag tac acc cga cgg cag aac agc gcc 1728
Thr His Thr Ile Ser Gly Leu Gln Tyr Thr Arg Arg Gln Asn Ser Ala
                565                 570                 575

aac gag cag gtc gcg gac tac gag atc tac acc agc ctg aac ggc acg 1776
Asn Glu Gln Val Ala Asp Tyr Glu Ile Tyr Thr Ser Leu Asn Gly Thr
            580                 585                 590

acc tgg gat ggc ccg gtt gcc agc ggg cgc ttc acc acg tcc ctc gcg 1824
Thr Trp Asp Gly Pro Val Ala Ser Gly Arg Phe Thr Thr Ser Leu Ala
        595                 600                 605

ccg cag cgc gcg gtc ttc ccg gcg cgg gac gcc agg tac atc cgg ttg 1872
Pro Gln Arg Ala Val Phe Pro Ala Arg Asp Ala Arg Tyr Ile Arg Leu

    610                 615                 620

gtg gcc ctc agc gag cag acc ggg cac aag tac gcc gcg gtc gct gag 1920
Val Ala Leu Ser Glu Gln Thr Gly His Lys Tyr Ala Ala Val Ala Glu
625                 630                 635                 640

ctg gag gtg gaa ggc cag cgc 1941
Leu Glu Val Glu Gly Gln Arg
                645

<210> 2
<211> 647
<212> PRT
<213> Micromonospora viridifaciens

<400> 2

Met Thr Ala Asn Pro Tyr Leu Arg Arg Leu Pro Arg Arg Arg Ala Val
1               5                   10                  15

Ser Phe Leu Leu Ala Pro Ala Leu Ala Ala Ala Thr Val Ala Gly Ala
            20                  25                  30

Ser Pro Ala Gln Ala Ile Ala Gly Ala Pro Val Pro Pro Gly Gly Glu
        35                  40                  45
Pro Leu Tyr Thr Glu Gln Asp Leu Ala Val Asn Gly Arg Glu Gly Phe
    50                  55                  60
Pro Asn Tyr Arg Ile Pro Ala Leu Thr Val Thr Pro Asp Gly Asp Leu
65                  70                  75                  80
Leu Ala Ser Tyr Asp Gly Arg Pro Thr Gly Ile Asp Ala Pro Gly Pro
                85                  90                  95
Asn Ser Ile Leu Gln Arg Arg Ser Thr Asp Gly Gly Arg Thr Trp Gly
            100                 105                 110
Glu Gln Gln Val Val Ser Ala Gly Gln Thr Thr Ala Pro Ile Lys Gly
        115                 120                 125
Phe Ser Asp Pro Ser Tyr Leu Val Asp Arg Glu Thr Gly Thr Ile Phe
    130                 135                 140

Asn Phe His Val Tyr Ser Gln Arg Gln Gly Phe Ala Gly Ser Arg Pro
145                 150                 155                 160

Gly Thr Asp Pro Ala Asp Pro Asn Val Leu His Ala Asn Val Ala Thr
                165                 170                 175

Ser Thr Asp Gly Gly Leu Thr Trp Ser His Arg Thr Ile Thr Ala Asp
            180                 185                 190

Ile Thr Pro Asp Pro Gly Trp Arg Ser Arg Phe Ala Ala Ser Gly Glu
        195                 200                 205

Gly Ile Gln Leu Arg Tyr Gly Pro His Ala Gly Arg Leu Ile Gln Gln
    210                 215                 220

Tyr Thr Ile Ile Asn Ala Ala Gly Ala Phe Gln Ala Val Ser Val Tyr
225                 230                 235                 240

Ser Asp Asp His Gly Arg Thr Trp Arg Ala Gly Glu Ala Val Gly Val
                245                 250                 255
Gly Met Asp Glu Asn Lys Thr Val Glu Leu Ser Asp Gly Arg Val Leu
            260                 265                 270
Leu Asn Ser Arg Asp Ser Ala Arg Ser Gly Tyr Arg Lys Val Ala Val
        275                 280                 285
Ser Thr Asp Gly Gly His Ser Tyr Gly Pro Val Thr Ile Asp Arg Asp
    290                 295                 300
Leu Pro Asp Pro Thr Asn Asn Ala Ser Ile Ile Arg Ala Phe Pro Asp
305                 310                 315                 320
Ala Pro Ala Gly Ser Ala Arg Ala Lys Val Leu Leu Phe Ser Asn Ala
                325                 330                 335
Ala Ser Gln Thr Ser Arg Ser Gln Gly Thr Ile Arg Met Ser Cys Asp
            340                 345                 350

Asp Gly Gln Thr Trp Pro Val Ser Lys Val Phe Gln Pro Gly Ser Met
        355                 360                 365
Ser Tyr Ser Thr Leu Thr Ala Leu Pro Asp Gly Thr Tyr Gly Leu Leu
    370                 375                 380
Tyr Glu Pro Gly Thr Gly Ile Arg Tyr Ala Asn Phe Asn Leu Ala Trp
385                 390                 395                 400
Leu Gly Gly Ile Cys Ala Pro Phe Thr Ile Pro Asp Val Ala Leu Glu
                405                 410                 415
Pro Gly Gln Gln Val Thr Val Pro Val Ala Val Thr Asn Gln Ser Gly
            420                 425                 430
Ile Ala Val Pro Lys Pro Ser Leu Gln Leu Asp Ala Ser Pro Asp Trp
        435                 440                 445

Gln Val Gln Gly Ser Val Glu Pro Leu Met Pro Gly Arg Gln Ala Lys
    450                 455                 460
Gly Gln Val Thr Ile Thr Val Pro Ala Gly Thr Thr Pro Gly Arg Tyr
465                 470                 475                 480
Arg Val Gly Ala Thr Leu Arg Thr Ser Ala Gly Asn Ala Ser Thr Thr
                485                 490                 495
Phe Thr Val Thr Val Gly Leu Leu Asp Gln Ala Arg Met Ser Ile Ala
            500                 505                 510
Asp Val Asp Ser Glu Glu Thr Ala Arg Glu Asp Gly Arg Ala Ser Asn
        515                 520                 525
Val Ile Asp Gly Asn Pro Ser Thr Phe Trp His Thr Glu Trp Ser Arg
    530                 535                 540
Ala Asp Ala Pro Gly Tyr Pro His Arg Ile Ser Leu Asp Leu Gly Gly
545                 550                 555                 560

Thr His Thr Ile Ser Gly Leu Gln Tyr Thr Arg Arg Gln Asn Ser Ala
                565                 570                 575

Asn Glu Gln Val Ala Asp Tyr Glu Ile Tyr Thr Ser Leu Asn Gly Thr
            580                 585                 590

Thr Trp Asp Gly Pro Val Ala Ser Gly Arg Phe Thr Thr Ser Leu Ala
        595                 600                 605

Pro Gln Arg Ala Val Phe Pro Ala Arg Asp Ala Arg Tyr Ile Arg Leu
    610                 615                 620

Val Ala Leu Ser Glu Gln Thr Gly His Lys Tyr Ala Ala Val Ala Glu
625                 630                 635                 640

Leu Glu Val Glu Gly Gln Arg
                645